# EUROPÄISCHE PATENTSCHRIFT

(11) **EP 0 983 095 B1**
(45) Veröffentlichungstag und Bekanntmachung des Hinweises auf die Patenterteilung: **29.09.2004**
(21) Anmeldenummer: 98925574.0
(22) Anmeldetag: 09.05.1998
(51) Int. Cl.: A61L 27/00, C08L 89/00, C07K 17/06

(54) **PEPTID-BESCHICHTETE IMPLANTATE UND VERFAHREN ZU IHRER HERSTELLUNG**
PEPTIDE-COATED IMPLANTS AND METHODS FOR PRODUCING THEM
IMPLANTS RECOUVERTS DE PEPTIDES ET PROCEDE PERMETTANT DE LES PREPARER

(30) Priorität: 22.05.1997 DE 19721352; 16.12.1997 DE 19755801; 23.04.1998 DE 19818098
(43) Veröffentlichungstag der Anmeldung: 08.03.2000
(73) Patentinhaber: Biomet Deutschland GmbH, 14167 Berlin (DE)
(72) Erfinder: KESSLER, Horst, D-85748 Garching (DE); FINSINGER, Dirk, D-85748 Garching (DE); JONCZYK, Alfred, D-64295 Darmstadt (DE); MEYER, Jörg, D-63329 Egelsbach (DE); NIES, Berthold, D-64407 Fränkisch-Crumbach (DE); KANTLEHNER, Martin, D-85354 Freising (DE)
(86) Internationale Anmeldenummer: PCT/EP1998/002753
(87) Internationale Veröffentlichungsnummer: WO 1998/052619

(56) Entgegenhaltungen:
- WO-A-92/00047
- HIRANO Y ET AL.: "Synthesis and cell attachment activity of bioactive oligopeptides: RGD, RGDS, RGDV, and RGDT" JOURNAL OF BIOMEDICAL MATERIALS RESEARCH, Bd. 25, Nr. 12, Dezember 1991, Seiten 1523-1534, XP002090255 in der Anmeldung erwähnt

## Beschreibung

Die Erfindung betrifft Implantate allgemeiner Art für den menschlichen und tierischen Körper, welche mit Peptiden beschichtet sind, die die Adhäsion spezifischer Zellen in der jeweiligen Umgebung des Implantats selektiv zu vermitteln vermögen. Insbesondere betrifft die Erfindung mit RGD-Peptiden beschichtete Implantate und Verfahren zu ihrer Herstellung.

Die Erfindung beruht auf dem Prinzip der zielgerichteten Adhäsionsstimulierung ausgewählter Zell-Spezies auf Oberflächenbeschichtungen von Biomaterialien im allgemeinen und Implantaten im speziellen zum Zweck der gewebeselektiven, beschleunigten und verstärkten Integration derselbigen nach operativem Einbringen in das entsprechende Gewebe.

Auf diese Weise können verschiedene Oberflächenteile eines Implantats mit verschiedenen, die Zelladhäsion vermittelnden Peptiden, insbesondere RGD-Peptiden beschichtet und zur Verfügung gestellt werden, die der speziellen Gewebeumgebung, in welcher die Implantate eingebracht werden, Rechnung tragen.

Auf diese Weise wird zudem im Hinblick auf "Tissue Engineering" die Generation "intelligenter" biohybrider Organe durch Selbstorganisation möglich, die die biologische Information zu einer Organregeneration durch gezielte Aktivierung verschiedener Zell-Spezies durch verschiedene Peptide in unterschiedlichen Regionen der Implantatoberfläche tragen.

Der Terminus "erfindungsgemäße Peptide" umfaßt im folgenden, wenn nicht speziell anderes oder zusätzliches gesagt wird, alle Peptide, die die Zelladhäsion zu vermitteln vermögen. Hierunter sind vor allem solche gemeint, die die Aminosäuren Arginin (R), Glycin (G) und Asparaginsäure (D) hintereinander enthalten (RGD-Peptide). Beispiele von geeigneten RGD-Peptiden und geeigneten nicht RGDhaltigen Peptiden sind weiter unten aufgerührt. Umfaßt sind ferner entsprechende Peptide, die nicht die RGD-Sequenz enthalten, aber dennoch die Zelladhäsion beeinflussen. Im weitesten Sinne umfaßt die Erfindung auch nicht-peptidische Verbindungen, welche qualitativ die gleiche biologische Aktivität wie die besagten peptischen Verbindungen aufweisen.

Als Biomaterialien oder Implantate im erfindungsgemäßen Sinn werden Materialien bezeichnet, die in den menschlichen oder tierischen Körper eingebracht werden können, um die Funktion des entsprechenden funktionsgeschädigten natürlichen Gewebes wiederherzustellen. Hierzu zählen beispielsweise Hüftendoprothesen, künstliche Kniegelenke, Kieferimplantate, Sehnenersatz, Hautersatz, Gefäßprothesen, Herzschrittmacher, künstliche Herzklappen, Brustimplantate, Stents, Katheter und Shunts.

Das Integrationsverhalten von Implantaten in den Körper erweist sich nach wie vor als problematisch. Die Gewebeintegration der Materialien verläuft häufig zu langsam und zu unvollständig, um eine für die Funktionalität ausreichende mechanische Stabilität der Gewebe/Biomaterial-Bindung herzustellen. Ursächlich verantwortlich hierfür ist häufig die Beschaffenheit der Implantatoberfläche, die aufgrund ihrer unzureichenden Grenzflächenkompatibilität bzw. Bioverträglichkeit eine aktive Anlagerung von umliegendem gesunden Gewebe bzw. Zellen behindert. Dies erschwert die Ausbildung einer stabilen Gewebe-Implantat-Grenzschicht und führt damit zu einer inadäquaten Gewebeintegration, die wiederum in Lockerungen, Geweberesorption, Infektionen, Entzündungen, Allergien, Microthrombenbildung (Restenose) resultieren. In der Folge werden Revisionseingriffe zum Austausch der Implantate (z. B. Hüftendoprothesen, Kieferimplantate, Katheter oder externe Fixateure) und damit erneute operative Eingriffe erforderlich (Malchau und Herberts, 1996, Prognosis of the Total Hip Arthroplasty, 63. Annual Meeting of the American Academy of Orthopaedic Surgeons, Atlanta; Haddad et al, 1996, The Journal of Bone and Joint Surgery, 78-B:546-549; Collinge et al., 1996, Pin Tract Infections).

Darüber hinaus erweist sich insbesondere bei Hüftendoprothesen die sogenannte aseptische Implantatlockerung als problematisch, bei der nicht, wie gewünscht, Knochenzellen und damit Knochengewebe die direkte Verbindung zum Biomaterial ausbilden, sondern als störende Elemente Fibroblasten und Bindegewebe auftreten. In der Konsequenz wird die Prothese dadurch anstelle von Knochengewebe von Bindegewebe umkleidet, wobei die resultierende Stabilität der Prothese-Bindegewebe-Bindung nicht ausreicht, um den mechanischen Anforderungen an die Kraftübertragung eines künstlichen Hüftgelenks gerecht zu werden. Dies kann in der Folge zu einer Auslockerung der Prothese führen (Pilliar et al., 1986, Clin. Orthop., 208:108-113) und erfordert ebenfalls eine Revision. Ein weiteres Beispiel für eine Anhaftung unerwünschter Zelltypen auf Implantaten stellen Blutplättchen dar, die zur Ausbildung von Microthrombi und damit zu einer gestörten Implantatintegration führen können (Phillips et al., 1991, Cell 65, 359).

Besonders schwerwiegend wirkt der Mangel an Integrierbarkeit von Biomaterialien bzw. Implantaten in den Körper im Falle von kompletten Ersatzorganen, da hier die verschiedenartigen Zelltypen in Kontakt mit dem Implantat treten und die erforderliche Integrierbarkeit zielgerichtet sein sollte. Um extrem aufwendige Transplantationsprozeduren mit Hilfe anderer Patienten zu vermeiden, wird beispielsweise angestrebt, die Therapie des Funktionsversagens von Leber, Pankreas, Niere und Milz immer häufiger im Rahmen des "Tissue Engineering" mittels sogenannter biohybrider Organe zu vollführen, die aus Trägermaterialien bestehen, welche mit lebenden Zellen belegt sind und als funktionelle Einheit implantiert werden können. Meistens werden hierzu funktionsfähige, gesunde Zellen in vitro in resorbierbare oder nicht resorbierbare Membranen eingeschlossen bzw. verkapselt und als künstliche biohybride Organe oder Hohlorgane in den Patienten transplantiert (z. B.: Lim et al., 1980, Science 210, 908-912; Altman et al., 1982, Horm. Met. Res. Suppl. 12, 43-45; Zekorn et al., 1989, Transplantation Proceedings 21, 2748-2750; Altman et al., 1982, Horm. Met. Res. Suppl. 12, 43-45; EP 0 504 781 B1). Allerdings treten auch hier sehr oft die beschriebenen Problematiken der fibrösen Einscheidung mit verbundener mangelnder Nährstoffversorgung der Transplantate, immunologischer Abwehrreaktionen durch Zell-Freisetzung aus den Kapseln sowie die Bildung von Blutgerinnseln aufgrund der Thrombogenität der Materialoberflächen auf.

Es ist bekannt, die Gewebeintegration von Biomaterialien/ Implantaten durch Beschichtung derselben mit Peptiden, die die Zell-Adhäsion vermitteln, zu stimulieren. Hierzu kennt man zum einen solche Peptide, die die Tripeptid-Aminosäure-Sequenz Arginin-Glycin-Asparaginsäure (RGD), bzw. deren nicht-peptidische Analoga und zum anderen zelladhäsionsvermittelnde, nicht RGD-haltige Peptide (Beispiele siehe unten), bzw. deren nicht-peptische Analoga enthalten, welche bekanntermaßen als integrale Bestandteile vieler Proteine u. a. der extrazellulären Matrix (z. B. Kollagen Typ I, Fibronectin, Laminin, Vitronectin, Entactin, Osteopontin, Thrombospondin) oder der Blutgerinnungskaskade (Fibrinogen, von Willebrand Faktor) als zentrale Erkennungsmuster für die Adhäsion von eukaryontischen Zellen fungieren (z.B.: Pierschbacher und Ruoslahti, 1984, Nature, 309:30-33; Yamada, 1991, J. Biol. Chem., 266:12809-12812). Die erfindungsgemäß definierten Sequenzen werden von den jeweiligen Rezeptoren auf der Zell-Oberfläche, den Integrinen, erkannt und gebunden. Da die Adhäsion von Zellen an die entsprechenden Proteine durch eine Vielzahl verschiedener Integrine vermittelt wird, ist das Integrin-Expressionsmuster einer Zell-Spezies entscheidend für deren Adhäsionseigenschaften an diese Proteine. Das maßgeschneiderte Design und die Synthese von zumeist kurzkettigen, mit den entsprechenden Sequenzen ausgestatteten Peptiden, die selektiv und spezifisch nur an bestimmte Integrine binden können, ermöglicht die zielgerichtete Aktivierung nur derjenigen Zell-Spezies, die diese Integrine exprimieren. So kennt man z. B. RGD-Peptide, die selektiv an alphaᵥ-Integrin-Rezeptoren binden und damit bevorzugt die Bindung (Adhäsion) von alphaᵥbeta₃- / alphaᵥbeta₅- tragende Zellen (Osteoblasten, Osteoclasten, Endothelzellen) stimulieren können, ohne gleichzeitig die Adhäsion von unerwünschten Zellspezies, z.B. α_{IIb}β₃-tragende Blutplättchen stimulieren zu können (Haubner et al., 1996, 7, Am. Chem. Soc., 118:7461 ). Andere RGD-Peptide zeigen dagegen eine umgekehrte Wirkung und binden bevorzugt an α_{IIb}β₃- Integrinrezeptoren, so daß sie eine Selektivität z. B. für Blutplättchen aufweisen (Phillips et al., 1991, Cell 65, 359).

Die Beschickung von Implantatoberflächen mit synthetisch zugänglichen, erfindungsgemäß definierten Peptiden ist bekannt. Dabei werden die Peptide entweder durch Adsorption oder aber durch kovalente Bindung auf der Oberfläche mehr oder weniger fixiert. In der DE 1 97 06 667 werden zum Beispiel Biomaterialien beschrieben, die Knochenersatzmaterialien betreffen, die auf einem porösen Polymermaterial basieren, das eine durch Adsorption bedingte Oberflächenbelegung mit Peptiden mit RGD-Aminosäuresequenz aufweist. Aus der WO 91-05036 sind weiterhin metallische Prothesen, insbesondere aus Titan bzw. Titanlegierungen bekannt, an deren Oberflächen Peptide, die u.a. auch RGD-Sequenzen aufweisen können, kovalent gebunden sind. Valentini et al. (May 1997, Transactions of the 23rd Annual Meeting of the Society for Biomaterials, New Orleans, USA) beschreiben die Kovalente Anbindung von RGD-Peptiden an mit einer fluorinierten Ethylenpropylen-Zwischenschicht versehene Titanschrauben. Rezania et al. berichten auf der gleichen Veranstaltung von Siliciumdioxid- bzw. Titandioxid-Oberflächen welche über kovalente Bindung mit aminofunktionalen Organosilanen beschichtet sind und vollführen mittels eines heterobifunktionalen Quervernetzers wiederum kovalent die Anbindung thiolhaltiger RGD-Peptide.

Diese technischen Lösungen gehen jedoch nicht auf das Erfordernis ein, Implantate bzw. Biomaterialien zur Verfügung zu stellen, deren Oberfläche gezielt mit erfindungsgemäß definierten Peptiden beschichtet sind, die selektiv an den jeweiligen Zelltyp des das betreffende Implantat umgebenden Gewebes angepaßt sind.

Es wäre daher wünschenswert, Biomaterialien in der Art verändern zu können, daß gezielt diejenigen Gewebe oder Zell-Spezies, die auch nach Einbringen des Implantats in den Körper mit diesem aktiv in Funktion treten sollen, also z. B. Knochenzellen bei Hüftendoprothesen oder Epithelzellen für Haut-, Haar- oder Zahnersatz, ausschließlich oder bevorzugt zu deren Gewebeintegration veranlaßt werden, während gleichzeitig diesen Prozeß störende Zell-Spezies, wie z. B. Blutplättchen oder Fibroblasten, die die Ausbildung von Microthrombi bzw. Bindegewebskapseln fördern, an einer selektiven Wechselwirkung mit dem Implantat behindert werden sollen.

Es wäre ferner eine wünschenswerte und attraktive Strategie, Implantate mit denjenigen erfindungsgemäß definierten Peptiden (bzw. deren nicht-peptidischen Analoga) zu beschichten, die die Adhäsion derjenigen ausgewählten Zelltypen ausschließlich oder zumindest bevorzugt stimulieren, welche die entsprechenden komplementären Integrine tragen, was in der Folge zur beschleunigten in vivo-Synthese des entsprechenden ausgewählten Gewebes führt.

Hinsichtlich der Entwicklung kompletter biohybrider Organe (Haut, Blutgefäße, Harnwege, Blase, Ösophagus, Pankreas, Leber, Milz, Niere) wäre es ein entscheidender Fortschritt, die jeweils für ein bestimmtes Organ gewünschten verschiedenen Zell-Spezies durch Beschichtung unterschiedlicher Oberflächenteile eines Implantats mit verschiedenen zellselektiven erfindungsgemäß definierten Peptiden zielgerichtet, räumlich definiert und koordiniert zur Durchführung unterschiedlicher zellulärer in vivo-Prozesse aktivieren zu können.

Die vorliegende Erfindung beschreibt nun die Möglichkeit der Biofunktionalisierung von Biomaterialien, insbesondere Implantaten für alle denkbaren Organe durch deren Beschichtung mit synthetisierten zell- bzw. gewebeselektiven erfindungsgemäß definierten RGD-Peptiden, die in vitro die Adhäsion vorwiegend derjenigen Zell-Spezies stimulieren, die jeweils die Gewebeintegration des entsprechenden Biomaterials vollführen sollen und die gleichzeitig in vitro die Adhäsion vorwiegend derjenigen Zell-Spezies, die diesem Prozeß entgegenwirken, nicht stimulieren. Mit der Verwendung solcher Beschichtungen ist eine beschleunigte und die verstärkte Integration verschiedener Biomaterialien/Implantate mit verbesserter Langzeitstabilität nach deren Einbringen in den Körper zu erzielen.
Darüber hinaus bestehen mit diesem Konzept der Beschichtung verschiedener Materialoberflächenteile eines Implantats mit unterschiedlichen erfindungsgemäß definierten Peptiden alle Möglichkeiten zur Entwicklung von "intelligenten", biohybriden Organen ("Tissue Engineering"), die die biologisch Information zur selektiven Aktivierung verschiedener Zielgewebe bzw. Zielzellen tragen und damit durch Selbstorganisation in den Körper integriert werden und hierdurch eine Gewebeintegration verstärken oder gar erst ermöglichen können.

Gegenstand der Erfindung ist somit ein Implantat, welches geeignet für unterschiedliche menschliche und tierische Organe ist, bestehend im wesentlichen aus einer Trägermatrix und einer diese Matrix umhüllenden Peptid-Beschichtung, die zur zielgerichteten Adhäsionsstimulierung von menschlichen oder tierischen Körperzellen verschiedene oder gleichartige Peptide enthält, welche Sequenzen aufweisen, die Bindungsstellen auf den für die Adhäsion verantwortlichen Integrin-Rezeptoren auf humanen oder tierischen Zellen erkennen, wobei die Trägermatrix reaktive bindungsfähige Gruppen an ihrer Oberfläche aufweist, die dazu befähigt sind mit entsprechenden funktionellen reaktiven Gruppen besagter Peptidschicht eine stabile kovalente Bindung einzugehen, dadurch gekennzeichnet, dass beragte auf der Oberfläche des Implantats angeordnete Peptide so ausgewählt und lokal angeordnet sind, daß sie aufgrund ihrer entsprechend unterschiedlichen strukturbedingten, zelladhäsions-stimulierenden Aktivität spezifisch dem natürlichen unterschiedlichen komplementären Integrinmuster der in jeweiligen Region an sie angrenzenden Gewebezellen entsprechen, in die das Implantat eingebracht werden soll, wodurch ein lokal differenziertes und selektives bioaktives Beschichtungsmuster der Implantatoberfläche vorliegt.

Gegenstand der Erfindung ist ferner ein Verfahren zur Herstellung von für Organe/Gewebe geeigneten Implantaten auf Basis einer anorganischen Trägermatrix, die eine Oberfläche besitzen, welche mit die Zelladhäsion stimulierenden Peptiden beschichtet sind, wobei die besagten Peptide selektiv in Bezug auf das komplementäre Integrinmuster der an das Implantat unmittelbar angrenzenden Gewebezellen sind, das dadurch gekennzeichnet ist, daß man nach an sich bekannten Methoden (i) in vitro die Integrinrezeptor-Struktur der Zielzellen bzw. des Zielgewebes, in welches das Implantat in vivo eingebracht werden soll, bestimmt, (ii) die Peptide mit der entsprechenden komplementären Struktur auswählt bzw. synthetisiert und (iii) die besagten Peptide an die betreffende Oberfläche des Implantats bindet.
Insbesondere sind Gegenstand der Erfindung Verfahren und Implantate/Biomaterialien mit folgenden Charakteristika: die besagten Peptide, insbesondere RGD-Peptide sind durch kovalente Bindung, gegebenenfalls über verzweigte, oberflächenvergrößernde Moleküle und/oder Molekülanker an die Implantatoberfläche fixiert; bevorzugt werden RGD-Peptide verwendet, die alphaᵥbeta₃-/alphaᵥbeta₅tragende Zellen, insbesondere also beispielsweise die Adhäsion von Osteoblasten, Osteoclasten, Endothelzellen stimulieren können und gleichzeitig die Adhäsion von Blutplättchen oder Fibroblasten nicht zu stimulieren in der Lage sind; als Trägermatrices werden geformte oder ungeformte Teile aus Keramik, Polymermaterial oder Metall oder ein biohybrides Organ oder Hohlorgan eingesetzt.

Die erfindungsgemäß definierten Peptide sind auf molekularer Ebene im wesentlichen aus folgenden Bestandteilen konzipiert:
- einer die für die Adhäsion zuständige Aminosäuresequenz-tragenden Domäne (z.B. die genannte RGD-Sequenz), die selektiv eine ausgewählte Zell-Spezies erkennt und bindet.
- einem Abstandshalter, um die zellerkennende und die Erkennungssequenztragende Domäne den Zellen in solcher Art zu präsentieren, daß eine Zellbindung unter sterischen Gesichtspunkten erst möglich wird.
- einem Molekülanker, der die stabile Anbindung des betreffenden Peptid-Derivats an die Biomaterial- bzw. Implantatoberfläche vollzieht.
- optional kann die Zell-Adhäsion durch zusätzliche Kopplung der erfindungsgemäß definierten Peptide an verzweigte Molekülstrukturen (sogenannte Dendrimere oder Tentakel) die einen oberflächenvergrößernden Effekt ausüben, bevor die Bindung an die Biomaterialoberfläche erfolgt, gesteigert werden.

Unter der Oberfläche des Biomaterials bzw. Implantats ist erfindungsgemäß nicht nur die unmittelbare Oberfläche der Trägermatrix zu verstehen, sondern auch eine gegebenenfalls vorhandene zusätzliche Beschichtung aus beispielsweise polymerem Material, natürlichen oder artifiziellen Knochenmaterialien, Proteinen oder Proteinderivaten.
Als Trägermatrix eigenen sich vor allem Materialien aus Keramik, Metall, Polymermaterialien (z.B. PMMA) oder vorzugsweise resorbierbaren Knochenersatzmaterialien. Besonders geeignet sind resorbierbare bzw. bio-abbaubare Werkstoffe, z. B. aus Polylactiden, insbesondere racemische D,L Polylactid-Verbindungen oder resorbierbare Calciumphosphat-bzw. Hydroxyl-apatit- Mischungen, die bewirken können, daß der ursprüngliche Gewebezustand wiederhergestellt werden kann und wie sie beispielsweise aus der WO 96/36562 oder der EP 0 543 765 bekannt sind. Je nach Einsatzgebiet kann als Trägermatrix auch Collagen oder Agar geeignet sein.

Unter dem Begriff "biohybrides Organ" ist eine üblicherweise anorganische Matrix zu verstehen, die mit lebenden Zellen auf irgendeine Weise beladen bzw. verbunden sind (siehe oben). Erfindungsgmäß ist darunter auch eine entsprechende Anordnung zu verstehen, die frei von Zellen ist und nur die entsprechenden verschiedenartigen erfindungsgemäß definierten Peptide auf unterschiedlichen Implantat-Oberflächenteilen aufweist, welche, in das defekte Gewebe eingebracht, selektiv die umgebenden Zellen zu aktivieren vermögen. Der Vorteil solcher azellulärer biohybrider Organe besteht darin, daß "intelligente", biokompatible Implantate, die kostengünstig und kontrollierbar produziert werden können, die biologische Information zu einer Organregeneration tragen. Die Integration solcher biohybriden Organe in den Körper wird dann durch körpereigene Regenerationsprozesse mittels Selbstorganisation vollzogen, wodurch immunologische Abwehrreaktionen, wie sie beispielsweise durch implantierte Fremd-Zellen oder Fremd-Proteine oft auftreten, vermieden werden können.

Die Implantate liegen erfindungsgemäß in der Regel in Formkörpern bzw. Prothesen vor, wobei der Formkörper dem jeweiligen Gewebe- / Knochendefekt angepaßt sein sollte. Im Falle biohybrider Organe können die Prothesen lediglich aus mit oder ohne entsprechende Zellen und den erfindungsgemäß definierten Peptiden beschichteteten Membranen oder Folien bestehen oder aber die Anordnungen, wie sie z. B. aus der EP 0 504 781 bekannt sind, aufweisen.
Als erfindungsgemäß einsetzbare Peptide kommen alle Peptide und deren Verbindungen mit nichtpeptidischen Substituenten, die eine für die Zelladhäsion verantwortliche Domäne bzw. Aminosäuresequenz enthalten und die über ihre peptidischen und nichtpeptidischen Substituenten auf den Implantatoberflächen binden können, in Betracht. Insbesondere kommen entsprechende Peptide mit einer RGD-Sequenz in Frage.

Folgende Aufzählung von bevorzugten Peptiden bzw. Peptidverbindungen soll lediglich beispielhaften und keinerlei limitierenden Charakter haben, wobei folgende Abkürzungen verwendet werden:
- Asp (D) =: Asparaginsäure
- Gly (G) =: Glycin
- Arg (R) =: Arginin
- Tyr(Y) =: Tyrosin
- Ser(S) =: Serin
- Phe(F) =: Phenylalanin
- Lys (K) =: Lysin
- DPhe (f) =: D-Phenylalanin
- Pro(P) =: Prolin
- Leu(L) =: Leucin
- Ile(I) =: Isoleucin
- Val(V) =: Valin
- Glu(E) =: Glutaminsäure
- Thr(T) =: Threonin
- Ala(A) =: Alanin

*(a) Beispiele für geeignete RGD-haltige Peptide:*
   RGD (Arg-Gly-Asp),
   GRGD (Gly-Arg-Gly-Asp),
   GRGDY (Gly-Arg-Gly-Asp-Tyr),
   RGDS (Arg-Gly-Asp-Ser),
   GRGDS (Gly-Arg-Gly-Asp-Ser),
   RGDF (Arg-Gly-Asp-Phe),
   GRGDF (Gly-Arg-Gly-Asp-Phe),
   cyclo-RGDfK (Arg-Gly-Asp-DPhe-Lys),
   cyclo-RGDfKG (Arg-Gly-Asp-DPhe-Lys-Gly).
*(b) Beispiele für geeignete nicht RGD-haltige Peptide:*
   LDV (Leu-Asp-Val),
   LGTIPG (Leu-Gly-Thr-Ile-Pro-Gly),
   REDV (Arg-Glu-Asp-Val),
   IKVAV (Ile-Lys-Val-Ala-Val),
   YIGSRG (Tyr-Ile-Gly-Ser-Arg-Gly),
   LRE (Leu-Arg-Glu),
   PDSGR (Pro-Asp-Ser-Gly-Arg),
   DGEA (Asp-Gly-Glu-Ala),
   RYVVLPR (Arg-Tyr-Val-Val-Leu-Pro-Arg),

Die erfindungsgemäß definierten Peptide können sowohl linear als auch zyklisch sein. Die oben genannten Peptide bzw. Peptidsequenzen können auch innerhalb längerer Peptide mit je nach ausgewähltem erfindungsgemäßen Peptid etwa insgesamt 4 bis 20 Aminosäuren vorkommen. Ebenso sind Aminosäuren, welche D- oder L-Konfiguration aufweisen oder die C- und /oder N-alkyliert sind, erfindungsgemäß mit umfaßt. Unter zyklischen Peptiden werden erfindungsgemäß solche Peptide verstanden, die über eine Amidbindung ringförmig geschlossen sind, wobei im Molekül vorzugsweise keine freien Carboxyl- oder Amino-Gruppen vorhanden sind. Besonders bevorzugt sind erfindungsgemäß RGD-Peptide, insbesondere solche aus der oben genannten Liste und hiervon besonders das Pentapeptid RGDfK, welches in seiner zyklischen Form aus der DE-OS-1 95 38 741 bekannt und spezifisch für Osteoblasten ist sowie das ebenfalls in seiner zyclischen Form vorliegende Hexapeptid RGDfKG, das spezifisch für Thrombozyten ist.

Entsprechende erfindungsgemäß definierte lineare und zyklische Peptide sind zum Beispiel in folgenden Patentanmeldungen beschrieben. EP 0 632 053, EP 0 655 462, EP 0578 083, EP 0 770 622, DE 1 95 38 741. Insbesondere sind jene Peptide geeignet, die selektiv an alphaᵥbeta₃-/alphaᵥbeta₅-Integrin-exprimierende Zell-Spezies (z. B. Osteoblasten, Osteoclasten, Endothelzellen) binden, ohne gleichzeitig an z.B. α_{IIb}β₃-tragende Zellspezies (z.B. Blutplättchen) zu binden. Die Peptide sowie die entsprechenden Derivate können nach Standardmethoden leicht synthetisiert werden, sofern sie nicht anderweitig erhältlich sind.

Prinzipiell können die erfindungsgemäß definierten Peptide an die Oberfläche des Biomaterials durch Adsorption oder Kovalenzbindung fixiert sein. Die Adsorptionsmethode ist bei Verwendung unterschiedlicher Peptide an ein und demselben Implantat weniger gut geeignet, da die erfindungsgemäß lokal selektive unterschiedliche Belegung der Oberfläche mit dieser Technik nur schwer zufriedenstellend zu bewerkstelligen ist.

Die Kopplung der Peptide bzw. deren nicht-peptidische Analoga an Trägeroberflächen durch Kovalenzbindung meist mittels sogenannter Molekülanker ist per se hinreichend bekannt und beschrieben worden, so beispielsweise bei Singer et al. (1987, J.Cell.Biol. 104: 573); Brandley, Schnaar (1989, Develop. Biol. 135: 74); Massia, Hubbell (1990, Anal. Biochem. 187: 292); Hirano et al. (1991, J. Biomed. Mat. Res. 25: 1523); Lin et al. (1992, Biomaterials 13: 905); Nicol at al. (1992, J. Biomed. Mat. Res. 26: 393); Dee et al. (1995, Tissue Engin. 1: 135), ohne daß dabei allerdings auf die Beschichtung von Implantaten im allgemeinen und im speziellen in irgend einer Weise eingegangen wäre.

Gegenstand der vorliegenden Erfindung sind nun neue Anwendungen von an sich bekannten Beschichtungsmethoden zur Herstellung der erfindungsgemäßen Implantate, wie zum Beispiel das "Kevloc®"-Verfahren (EP 0 712 621), welches erstmals erfindungsgemäß zur Kopplung der genannten Peptide (bzw. deren nicht-peptidische Analoga) an Oberflächen, welche Acryolyl- bzw. Methacryolyl-Ankerkomponenten enthalten, eingesetzt wurde, oder das "Silicoater®"-Verfahren (DE-OS 42 25 106), das hier erfindungsgemäß zur Kopplung der entsprechenden Peptide mittels Acryloyl- oder Methacryloyl-Ankerkomponenten in der Regel über eine Acryloyl/Methacryloyl-Silanderivat-Zwischenschicht (z.B. 3-Methacryloxypropyl-trimethoxysilan) an die entsprechenden Trägermatrices verwendet wurde. Eine weitere Möglichkeit, die erfindungsgemäß definierten Peptide an die Oberfläche der Trägermatrix bzw. des Implantats zu binden, besteht in der analogen Anwendung eines Silanisierungsverfahrens, das in der DE-OS 43 21005 beschrieben ist, welches ursprünglich die technische Lehre der Beschichtung von Perlglanzpigmenten für Wasserlacksysteme für Metalle und Kunstoffe in der Automobil- und Kunsstoffindustrie darlegt. Ferner ist erfindungsgemäß ein Verfahren für die Beschichtung von Goldoberflächen mit thiolgruppentragenden Peptiden geeignet, das ursprünglich in einem anderen Zusammenhang beschrieben wurde (Heuvel et al., 1993, Analytical Biochem. 215: 223)

Die geschilderten Verfahren sind bislang für die Beschichtung von Implantaten zwecks deren Bioaktivierung noch nicht eingesetzt worden.

Die Kopplung der entsprechenden erfindungsgemäß definierten Peptide an die Implantatoberfläche erfolgt erfindungsgemäß über entsprechende Ankermoleküle, d.h. das Peptid wird in der Regel nicht unmittelbar selbst auf die Implantatoberfläche fixiert. Die Einfügung eines solchen, unten näher definierten Moleküls hat vor allem den Sinn, den sterischen Erfordernissen des biologischen Rezeptors auf den Zielzellen in Zusammenhang mit der Bindung des entsprechenden Peptids Rechnung zu tragen.
Die Implantatoberfläche muß hierzu entsprechende funktionelle Gruppen bzw. reaktive Einheiten tragen, welche eine Bindung der entsprechenden funktionellen Gruppe des Ankermoleküls ermöglichen. Die funktionellen Gruppen, die auf der Implantatoberfläche bereitzustellen sind, richten sich wiederum nach der Beschaffenheit der eigentlichen je nach Erfordernis unterschiedlichen Trägermatrix (Metall, Kunststoff, Knochenmaterialien). Bei Metallimplantaten, kann man beispielsweise eine für SH-Reste des Ankermoleküls reaktive Oberflächenschicht durch Bedampfung mit Gold erzeugen. Die Silanisierung von Metalloberflächen nach bekannten Verfahren (siehe oben) führt ebenfalls zu reaktiven Oberflächen, welche mit den geeigneten erfindungsgemäßen Ankermolekülen, ggf. unter Verwendung von silanhaltigen Haftvermittlern, Verbindungen eingehen können (siehe unten). Implantate aus natürlichen Knochen oder naturähnlichen Knochenmaterialien (z. B. Calciumphosphat-Zemente) können Ankermoleküle binden, welche eine reaktive Phosphonatgruppe enthalten (Prinzip beschrieben bei Chu, Orgel, 1997, Bioconjugates Chem. 8: 103). An Implantate aus Kunststoff auf Acrylatbasis (z. B. PMMA) oder aus anderen Materialien mit einer entsprechenden Kunststoffbeschichtung können wiederum erfindungsgemäße Ankermoleküle angekoppelt werden, die ihrerseits selbst einen reaktiven Acrylatrest aufweisen.

Ankermoleküle im Sinne der Erfindung sind also Moleküle at Basis von modifizierten bzw. substiutierten Alkylketten bzw. Kohlenstoffketten, die zumindest zwei unterschiedliche funktionelle Gruppe aufweisen, wobei eine funktionelle Gruppe in der Regel ein freier Carboxyl-Rest (freie NH₂-Gruppe) ist, der mit einer freien NH₂-Gruppe (freie Carboxylgruppe) einer Seitenkette eines erfindungsgemäß definierten Peptids, insbesondere eines RGD-Peptids, eine Amidbindung (-CO-NH-) erzeugt, und die andere funktionelle Gruppe, welche vorzugsweise am anderen Ende der C-Kette des Ankermoleküls lokalisiert ist und eine direkte oder indirekte Bindung mit der Implantatoberfläche bewirkt, je nach Beschaffenheit bzw. Erfordernis der Implantatoberfläche, vorzugsweise ein (meth)acrylathaltiger Rest oder eine Mercaptogruppe ist. Prinzipiell können auch andere funktionelle Gruppen verwendet werden, die mit den jeweiligen reaktiven Gruppen unmittelbar auf der Implantatoberfläche oder auf einer geeigneten Zwischenschicht zu einer stabilen Bindung zu reagieren vermögen.

Die Ankermoleküle der Erfindung besitzen, wie bereits angedeutet, gleichzeitig die Funktion von Abstandshaltern oder Spacem, weisen also neben ihren geschilderten Verknüpfungsoptionen eine entsprechende gegebenenfalls spezifisch angepaßte Länge auf, um zu ermöglichen, daß die für die Zelladhäsionsstimulierung verantwortliche Domäne den richtigen Abstand zur Zielzelle hat, so daß eine Zellbindung unter sterischen Gesichtspunkten verbessert oder erst ermöglicht werden kann.

Die biologische Funktion der zellerkennenden und die entsprechende Aminosäuresequenz-tragenden Domäne wurde beispielhaft anhand eines selektiv an alphaᵥbeta₃/alphaᵥbeta₅-Integrin-exprimierende Zell-Spezies (z. B. Osteoblasten, Osteoclasten, Endothelzellen) bindendes synthetisiertes Peptid bewiesen (Haubner et al., 1996, J. Am. Chem. Soc., 118:7461-7472).

Die Ankermoleüle der Erfindung weisen vorzugsweise folgende lineare Strukturen auf, wobei die erfindungsgemäß definierten Peptide über die NH₂-Gruppe einer ihrer Aminosäure-Seitenketten, vorzugsweise Lysin-Seitenkette an das freie Carboxyl-Ende des jeweiligen Ankermoleküls gebunden werden.
*(i) Mercapto(amido)carbonsäure-Derivate*:

   -CO-(CH₂)ₖ-X-SH,

   wobei X eine Einfachbindung oder -CO-NH-(CH₂)ₗ-,
   k = 2 bis 12 und l= 2 bis 4 bedeutet;
*(ii) Acrylamidocarbonsäure-Derivate:*

   -CO-(CH₂)ₘ-[NH-CO-(CH₂)ₙ]ₚ-NH-CO-CH=CH₂,

   wobei m, n = 2 bis 8; p = 0 bis 2 bedeuten,
*(iii) Acrylamido-amidotriethylenglycolsäure-Derivate*

   -(CO-CH₂-O-CH₂-CH₂-O-CH₂-CH₂-NH)_{q}-CO-(CH₂)ᵣ-NH-CO-CH=CH₂,

   wobei q = 1 bis 3 und r = 2 bis 8 bedeutet.

Bevorzugt sind insbesondere die folgenden Typen spezieller Ankermoleküle:
(ia) - CO-CH₂-CH₂-SH (Mercaptopropionsäure),
(ib) - CO-CH₂-CH₂-CO-N-H-CH₂-CH₂-SH (Mercaptoethylamidobernsteinsäure),
(iia) -CO-(CH₂)₅-NH-CO-CH=CH₂ (Acrylamidohexansäure),
(iib) -CO-(CH₂)₅-NH-CO-(CH₂)₅-NH-CO-CH=CH₂ (Acrylamidohexansäure-amidohexansäure),
(iiia) -CO-CH₂-O-CH₂-CH₂-O-CH₂-CH₂-NH-CO-(CH₂)₅-NH-CO-CH=CH₂ (Acrylamidahexansäure-amidotriethylenglycolsäure)
(iiib) -(CO-CH₂-O-CH₂-CH₂-O-CH₂-CH₂-NH)₂-CO-(CH₂)₅-NH-CO-CH=CH₂ (Acrylamidohexansäure-di-amidotriethylenglycolsäure).

Generell sind erfindungsgemäß jene Ankermolekülstrukturen bevorzugt, welche in der linearen C-Kette mindestens sechs C-Atome aufweisen. Es wurde nämlich überraschend gefunden, daß diese Länge des Ankermoleküls besonders günstig ist, um optimale Ergebnisse in bezug auf die beschleunigte und verstärkte Gewebeintegration des Implantats zu erzielen. Die Angabe von mindestens sechs C-Atomen in der linearen Kette bezieht sich erfindungsgemäß auf die Gesamtlänge des Moleküls zwischen Peptid und Implantatoberfläche. So sind auch Ankermoleküle der oben gezeigten Strukturen mit kürzerer Kette (z.B. Typ ia) geeignet, falls noch andere nicht genannte, kettenverlängernde Kopplungskomponenten zwischen Peptid und Implantatoberfläche eingefügt werden.

Die Ankermoleküle werden nach Standardmethoden mit den erfindungsgemäß definierten Peptiden über die Carboxyl-Funktion amidartig verbunden, wodurch Strukturen des Typs Peptid - NH-CO- Ankermolekül entstehen, welche wiederum, wie dargelegt, auf dem Implantat fixiert werden, wodurch wiederum Konstrukte des folgenden Typs entstehen:Peptid - NH - CO - Ankermolekül- Implantat(oberfläche). Bevorzugt sind entsprechende Implantat-Konstrukte, welche sich zusammensetzen aus einem der oben einzeln genannten definierten Peptide, insbesondere RGD-Peptide, einem der oben einzeln aufgeführen allgemeinen und speziell definierten Ankermoleküle und einem entsprechend oberflächenreaktiven Implantat. Besonders bevorzugt sind folgende Implantate:
cyclo-RGDfK NH-CO - Thiol-Derivate (Typ: i) - Implantat,
cyclo-RGDfK NH-CO - Acrylat-Derivate (Typ: ii) - Implantat,
cyclo-RGDfK NH-CO - Acrylat-Glycol-Derivate (Typ: iii) - Implantat,
cyclo-RGDfKG NH-CO - Acrylat-Glycol-Derivate (Typ: iii) - Implantat,
worin die lineare C-Kette des gesamten Ankermoleküls mindestens sechs C-Atome aufweist.

Darunter sind inbesondere bevorzugt:
cyclo-RGDfK NH-CO - Thiol-Derivat (Typ: ib) - Implantat
cyclo-RGDfK NH-CO - Acrylat-Derivat (Typ: iia) - Implantat
cyclo-RGDfK NH-CO - Acrylat-Derivat (Typ: iib) - Implantat
cyclo-RGDfK NH-CO - Acrylat-Glycol-Derivat (Typ: iiia) - Implantat
cyclo-RGDfKG NH-CO - Acrylat-Glycol-Derivat (Typ: iiia) - Implantat
cyclo-RGDfK NH-CO - Acrylat-Glycol-Derivat (Typ: iiib) - Implantat

Die Herstellung dieser bevorzugten Strukturen erfolgt nach Standardmethoden, bzw. ist weiter unten, oder in der am gleichen Tag angemeldeten Parallelanmeldung des Anmelders, die die Peptid-Ankerstrukturen als solche zum Gegenstand hat, beschrieben.

Wie bereits weiter oben angesprochen, werden zur Verankerung der beschriebenen zell- bzw. gewebeselektiven Peptid-Derivate an Biomaterialoberflächen gemäß der Erfindung im wesentlichen drei Alternativwege verfolgt, wobei das molekulare Erkennungsmuster der die jeweilige RGD-Sequenz tragenden Domäne selektiv für einen bestimmten Zelltyp sowie der Abstandshalter unverändert bleiben können, während der Molekülanker je nach den aufgeführten Kopplungsvarianten, variiert werden kann, beispielsweise durch:
- Kopplung von Thiol-Peptid-Derivaten an goldbeschichtete Biomaterialoberflächen (z.B. mit Typ (i) Ankermolekülen);
- Kopplung von (Meth)Acryloyl-Peptid-Derivaten an acrylat- bzw. methacrylat beschichtete Biomaterialoberflächen (z.B. mit Typ (ii) oder (iii) Ankermolekülen);
- Kopplung von (Meth)Acryloyl-Peptid-Derivaten an silanbeschichteten Biomaterial-oberflächen (z.B. mit Typ (ii) oder (iii) Ankermolekülen) mit einem (Meth)Acryloyl-Silan-derivat als Haftvermittler bzw. Zwischenschicht (z. B. 3-Methacryloxypropyl-trimethoxysilan).

Die Erzielung der kritischen Mindestlänge des Ankermoleküls für verschiedene Peptid-Kopplungsvarianten an Biomaterialoberflächen erfolgt durch Synthese der erfindungsgemäß definierten Peptide mit den erfindungsgemäß definierten Ankermolekülen von einer Kettenlänge mit vorzugsweise 6 bis 24 C-Atomen und wahlweise unterschiedlichen Hydrophobie- / Hydrophilie - Eigenschaften (z. B. durch Verwendung zahlenmäßig unterschiedlicher Einheiten von -CH₂- und / oder Amidohexansäure oder Ethylenglycol nach an sich Standardmethoden und anschließender Testung der biologischen Wirksamkeit durch Bestimmung der Zelladhäsion in vitro nach Beschichtung entsprechender Biomaterialoberflächen).

Auf die beschriebene Weise kann je nach den Materialeigenschaften des Implantats ein geeignetes Beschichtungsverfahren zur Konditionierung der Oberflächen vor der eigentlichen Kopplung mit den erfindungsgemäßen Peptid-Derivaten ausgewählt werden. Darüber hinaus ist in Abhängigkeit vom Gewebetyp bzw. dem Zelltyp, der die Integration des Biomaterials / Implantats vollführen soll, die Beschichtung mit anderen Peptiden möglich, die wiederum zielgerichtet die Integrine der entsprechenden Ziel-Zell-Spezies aktivieren, wie z. B. das alpha₆beta₄-Integrin von Epithelzellen (z. B. zum Einsatz von Knochen-, Kiefer-, Haut- oder Haarimplantaten) oder das alpha_{IIb}beta₃-Integrin von Blutplättchen. Alphaᵥbeta₃-spezifische RGD-Peptide weisen eine Selektivität gegenüber Endothelzellen und Osteoblasten auf, wodurch sie sich z. B. zur Beschichtung von Gefäßprothesen oder Knochenimplantaten eignen würden. Hierdurch kann für Implantate auf dem Gebiet des Knochen,-Gefäß, Zahn-, Haut- und Haarersatzes für nahezu jedes beliebige Organ eine geeignete bioaktivierende Oberflächenbeschichtung realisiert werden.

Ehe entsprechende erfindungsgemäße Implantate oder biohybride Organe bereitgestellt werden können, müssen zuvor die für den jeweiligen Zelltyp geeigneten Peptide in einem *in vitro*-Testsystem auf biologische Wirksamkeit geprüft und ermittelt werden, um später eine gezielte und selektive Beschichtung des Implantats, welches in das ausgesuchte Gewebe eingebracht werden soll, vornehmen zu können.

Die hierfür notwendige Analyse der Integrin-Rezeptor-Struktur des Zielgewebes bzw. der Zielzellen, in welches das Implantat eingebracht werden soll, erfolgt mittels gängiger, bekannter, immunhistologischer Verfahren, wie z. B. mittels Immunfluoreszenz oder der Immunhistochemie von Gewebeproben. Die hierfür erforderlichen Antikörper gegen verschiedene Integrin-Rezeptoren bzw. deren Untereinheiten sind mittlerweile bekannt und stehen zur Verfügung oder können entsprechend mittels an sich bekannter Standardmethoden, wie beispielsweise geeigneter Immunisierungen, erzeugt werden.

Die erfindungsgemäß definierten Peptide werden in verschiedenen Konzentrationen kovalent an Kulturoberflächen beispielsweise aus mit Rinderserumalbumin (BSA) beschichteten Polystyrol gekoppelt. Das Material dieses Testrägers spielt bei der Ermittlung der geeigneten Peptide keine essentielle Rolle. Ebenso ist die angewendete Kopplungsmethode hier noch von geringer Bedeutung. Aus praktischen Gründen kann die Kopplung bei diesen Ermittlungen auch mittels Inkubation und Adsorption der besagten Peptide an den Testträger erfolgen.
Anschließend wird die Adhäsion von ausgesuchten Gewebe-Zellkulturen (z. B. Osteoblasten), die den Zellen, die *in vivo* im natürlichen Gewebe aktiviert werden sollen, in ihren Adhäsionseigenschaften zu entsprechen vermögen, an die entsprechend beschichteten Oberflächen untersucht. Das Kriterium zur Auswahl geeigneter Zellkulturen für die Adhäsionsexperimente besteht im vergleichbaren Integrationsexpressionsmuster zu den Zielzellen *in vivo* nach Implantation, zum Beispiel deren alphaᵥbeta₃-/alphaᵥbeta₅- oder alpha_{IIb}beta₃-Expression, die mittels fluoreszenzmarkierten Antikörpern gegen alphaᵥbeta₃-, alphaᵥbeta₅- oder alpha_{IIb}beta₃- Integrine bzw. gegen die alphaᵥ-, alpha_{IIb}-, die beta₃- bzw. gegen die beta₅-Untereinheiten des Integrin-Rezeptors anhand eines Fluorescence Activated Cell Sorter (FACS) verifiziert wird. Bei anderen Ziel-Zell-Spezies *in vivo* mit unterschiedlichen Integrinrezeptor-Muster müssen entsprechend andere Antikörper eingesetzt werden. Solche sind mittlerweile bekannt und stehen zur Verfügung oder können entsprechend bekannter Standardmethoden z. B. mittels geeigneter Immunisierung erzeugt werden.

Die verschiedenen selektierten Zell-Spezies werden auf mit den in Frage kommenden Peptiden beschichteten, BSA-vorbehandelten Polystyrol-Kulturoberflächen angesät und inkubiert. Anschließend werden nicht adherierte Zellen abgewaschen.
Das Bindungsverhalten der unterschiedlichen, ausgewählten Zell-Spezies an den mit unterschiedlichen erfindungsgemäß definierten Peptiden beschichteten Testoberflächen entspricht im positiven Fall, das heißt, wenn eine ausreichende Spezifität vorliegt, jeweils einer Titrationskurve mit einer maximalen Adsorptionsrate von etwa 60 bis 100 % der angesäten Zellen sowie einer halbmaximalen Zellanbindung bei einer RGD-Peptid -Konzentration in der Beschichtungslösung von etwa 5nM bis 5µM.
In ähnlicher Weise, wie für die Kopplung geeigneter Peptide an BSAvorbeschichteten Polystyroloberflächen beschrieben, sind Verankerungsstrategien an modifizierte bzw. konditionierte Biomaterialoberflächen unter Verwendung verschiedener Haftvermittlungszwischenschichten möglich.

Zusammengefaßt, läßt sich folgendes sagen:
Die Implantate des Standes der Technik weisen folgende Nachteile auf:
- unvollständige, langsame Implantatintegration ins Gewebe,
- eingeschränkte Akzeptanz im Gewebe,
- unzureichende funktionelle Stabilität der Implantat/Gewebegrenzschicht
- mangelnde stimulierende Wirkung des Implantats auf die Gewebeneogenese
- unphysiologische Eigenschaften der Implantatoberfläche.
Als Konsequenzen hieraus ergaben sich weitere Probleme:
- aseptische Implantatlockerungen (z.B. fibröse Kapselbildung)
- lokale Bildung von Mikrothrombi,
- Infektionen,
- Entzündungen,
- Geweberesorptionen,
- Revisonen.

Diese Probleme können durch das bereitgestellte erfindungsgemäße Verfahren bzw. der hierdurch erzeugten Implantate weitgehend beseitigt werden. Die erfindungsgemäßen Gegenstände zeichnen sich aus durch:
- maßgeschneidertes Design von Adhäsionspeptiden, welche komplementär zum Integrin-Expressions- muster der Zielgewebe / Zielzellen sind;
- selektive Stimulierung der Zelladhäsion der Zielzellen, die die Gewebeneogenese vollführen sollen, ohne gleichzeitig die Adhäsion der Zellen, die den Prozeß behindern, zu adherieren;
- Beschichtungen höherer Stabilität mittels neuartiger Peptid-Anker-Moleküle;
- Beschleunigung und Verstärkung des Implantat-Integrationsprozesses in das Gewebe.

Es konnte gezeigt werden, daß der kritische sterische absolute Mindestabstand zwischen Zellerkennungssequenz auf dem Peptid und unbeschichteter Matarialoberfläche zwischen 2,0 und 3,5 nm, vorzugsweise zwischen 2,5 und 3,5 nm liegt. Maximale Belegungsraten (80 - 100%) können bei einem Mindestabstand von 3,0 bis 5,0 nm erzielt werden.

### Kurze Beschreibung der Abbildungen:

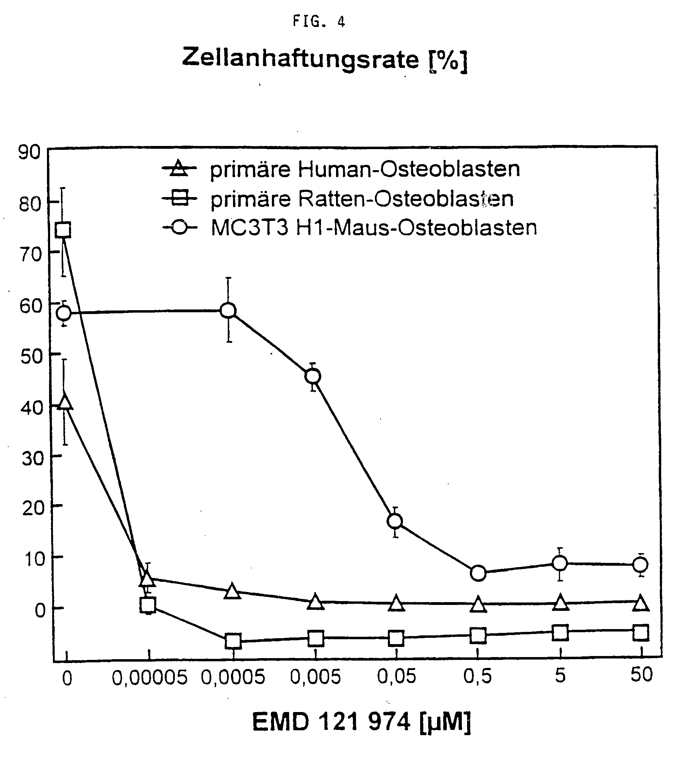

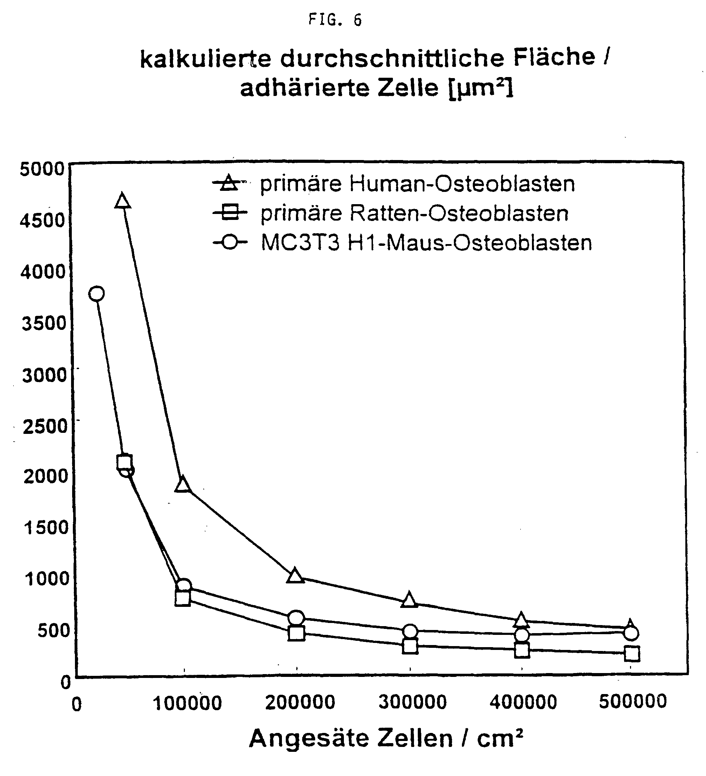

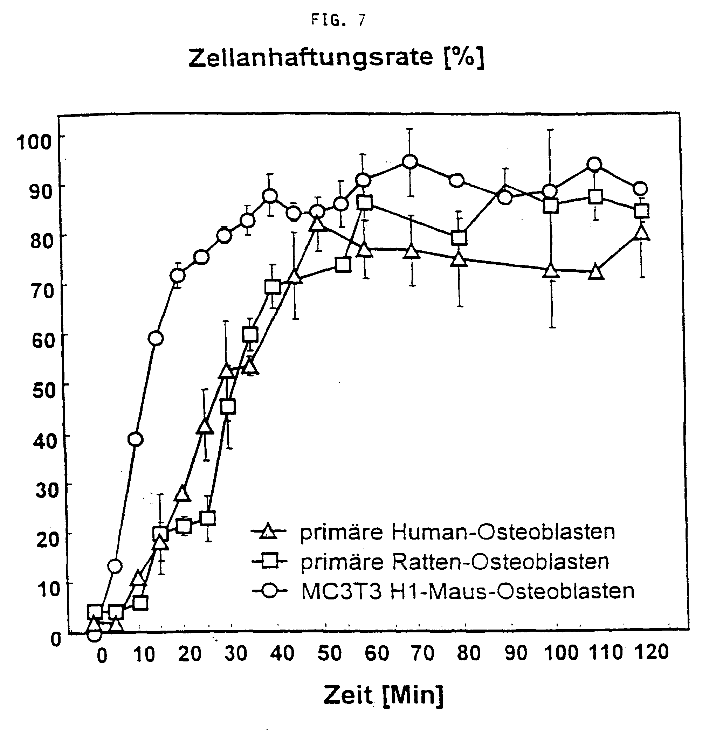

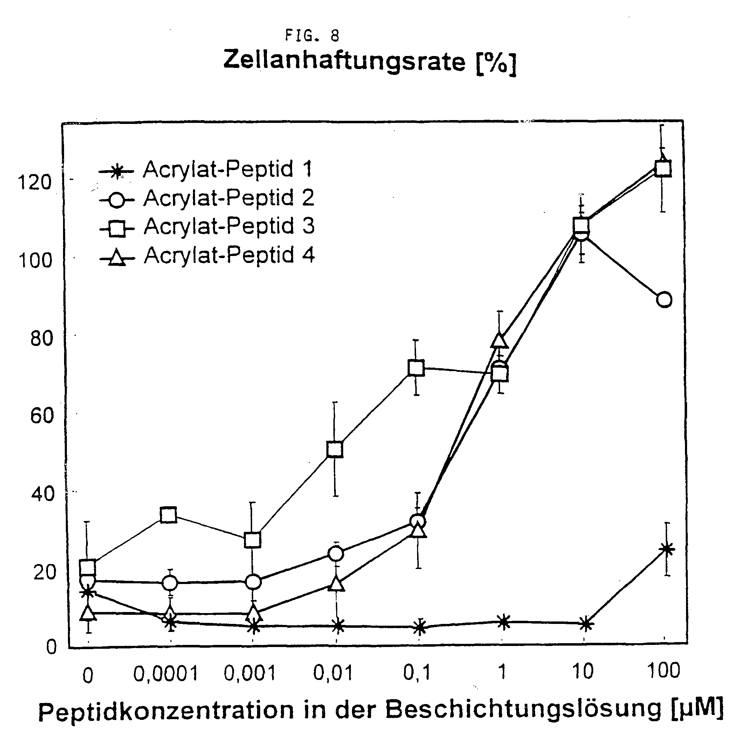

- Abb.1:: Analyse der Integrinzusammensetzung von primären Human-Osteoblasten durch FACS (Fluorescence Activated Cell Sorter) mittels fluoreszenzkonjugierter Antikörper gegen alphaᵥbeta₃- und alphaᵥbeta₅-Integrine.
x-Achse: Fluoreszenzintensität (Counts)
y-Achse: Zellzahl
M21: alphaᵥbeta₃- und alphaᵥbeta₅ - Positivkontrollen
M21: alphaᵥbeta₃- und alphaᵥbeta₅ - Negativkontrollen
HOB: Kultur primärer Human-Osteoblasten
ROB: Kultur primärer Ratten-Osteoblasten
- Abb. 2:: Adhäsion von MC3T3 H1-Osteoblasten an mit verschiedenen RGD-Peptiden über BSA beschichteten Polystyrol-Testoberflächen.
*x-Achse:* RGD-Peptid Konzentration in der Beschichtungslösung, (µM),
*y-Achse*: Zell-Belegungsgrad (%);
*obere Kurve*: cyclo-RGDfK NH - CO-CH₂-CH₂-S-(Sulfo-SMPB) ("Thiolpeptid 1-SMPB-Derivat"), *SMPB = Succinimidyl-4 (p-Maleimido-phenyl)-Butyrat;*
*mittlere obere Kurve*:cyclo-RGDfK NH-CO-CH₂-CH₂-CO-NH-CH₂-CH₂-S-(Sulfo-SMPB) ("Thiolpeptid 2-SMPB-Derivat")
*mittlere untere Kurve*: cyclo- RGDvE CO-NH-CH₂-CH₂-CH₂-CH₂-CH₂-CO-NH-CH₂-CH₂-S- (Sulfo SMPB) ("Thiolpeptid 3-SMPB-Derivat")
*untere Kurve*: Thiolpeptid-Kontrolle: cyclo-RβADfK-NH- CO-CH₂-CH₂-S-(Sulfo-SMPB)

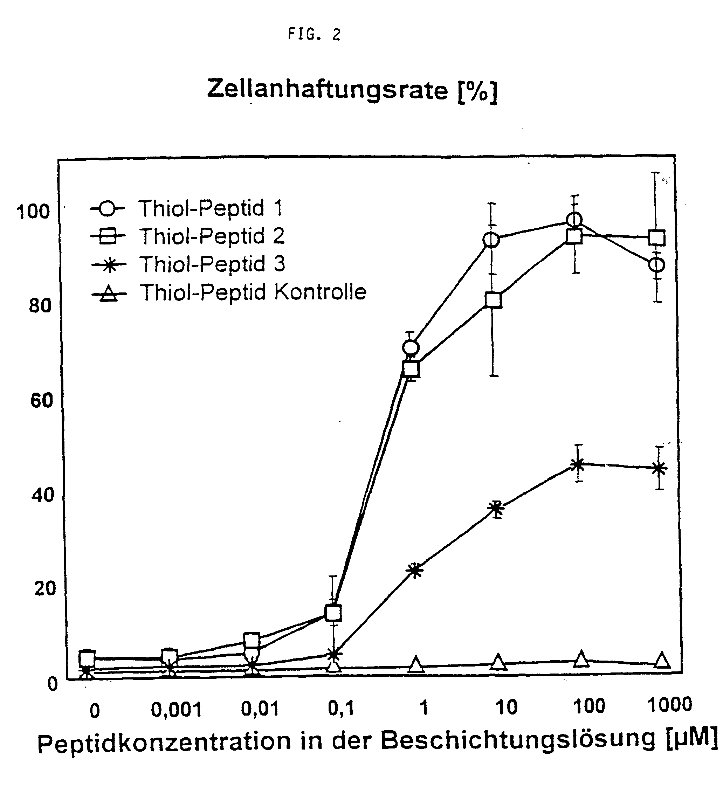

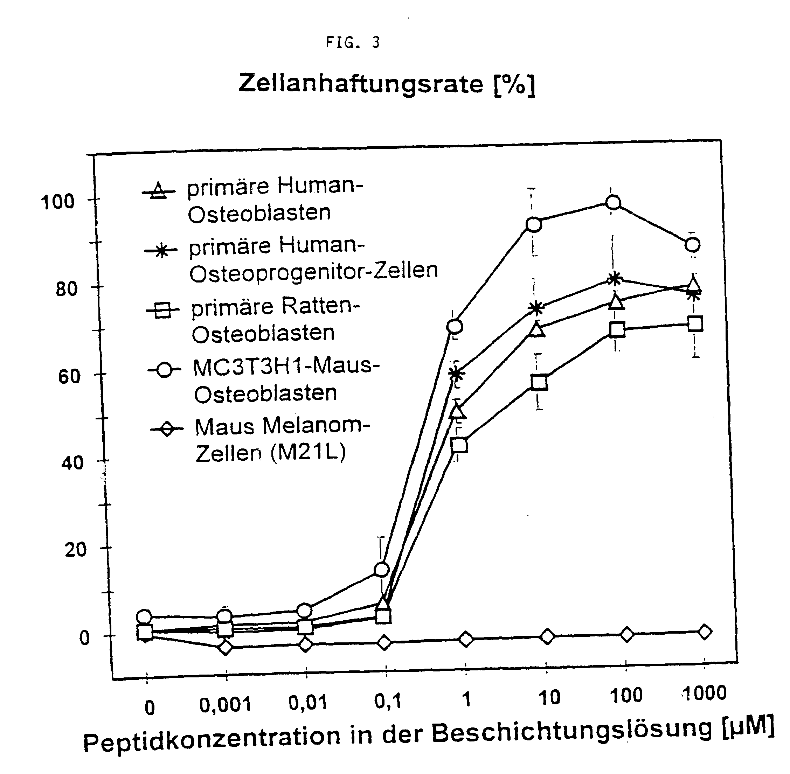

- Abb. 3:: Adhäsion von Osteoblasten an mit Thiolpeptid 1-SMPB-Derivat beschichteten Testoberflächen (s. unter Abb. 2):
x-Achse: Peptidkonzentration in der Beschichtungslösung (µM),
y-Achse: Zell-Belegungsgrad (%),
*obere Kurve:* MC3T3 H1-Mäuse-Osteoblasten;
*mittlere obere Kurve*: primäre humane Osteoprogenitorzellen
*mittlere Kurve:* primäre humane Osteoblasten
*mittlere untere Kurve*: alphaᵥbeta₃.Negativkontrollzellen M21L
*untere Kurve*: primäre Ratten-Osteoblasten
- Abb.4:: Adhäsion von Osteoblasten an mit Thiolpeptid 1-SMPP-Derivat (10 µM in der Beschichtungslösung) beschichteten Testoberflächen (s. Abb. 2)
x-Achse: Konzentr. an gelöstem cyclo-RGDfK (ohne Molekülanker) im Anhaftungsmedium (µM),
y-Achse: Zell-Belegungsgrad (%);
*obere Kurve*: MC3T3 H1-Mäuse-Osteoblasten;
*mittlere Kurve*: primäre Human-Osteoblasten
*untere Kurve*: primäre Ratten-Osteoblasten.

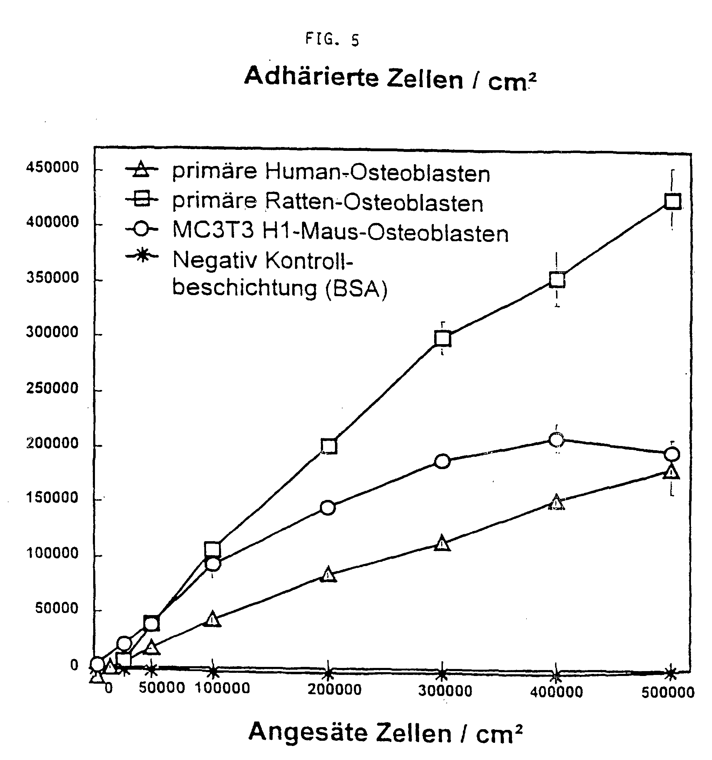

- Abb.5:: Adhäsion von Osteoblasten an mit Thiolpeptid 1-SMPB-Derivat (10 µM in der Beschichtungslösung) beschichteten Testoberflächen (s. Abb. 2),
x-Achse: Zahl angesäter Zellen / cm²
y-Achse: Zahl adherierter Zellen / cm²
*obere Kurve:* primäre Ratten-Osteoblasten
*mittlere obere Kurve:* MC3T3 H1-Mäuse-Osteoblasten;
*mittlere untere Kurve*: primäre Human-Osteoblasten
*untere Kurve:* BSA-Negativbeschichtung
- Abb.6:: Adhäsion von Osteoblasten an mit Thiolpeptid 1-SMPB-Derivat Derivat (10 µM in der Beschichtungslösung) beschichteten Testoberflächen (s. unter Abb. 2),
x-Achse: Zahl angesäter Zellen / cm²
y-Achse: berechneter Flächenbedarf / Zellen (µm²)
*obere Kurve:* primäre Human-Osteoblasten
*mittlere Kurve:* MC3T3 H1-Mäuse-Osteoblasten;
*untere Kurve:* primäre Ratten-Osteoblasten
- Abb.7:: Adhäsion von Osteoblasten an mit Thiolpeptid 1-SMPB-Derivat Derivat (10 µM in der Beschichtungslösung) beschichteten Testoberflächen (s. unter Abb. 2),
x-Achse: Zeit (min)
y-Achse: Zellbelegungsgrad (%)
*obere Kurve:* MC3T3 H1-Mäuse-Osteoblasten;
*mittlere Kurve:* primäre Human-Osteoblasten;
*untere Kurve:* primäre Ratten-Osteoplasten
- Abb.8:: Adhäsion von MC3T3 H1-Osteoblasten an mit verschiedenen RGD-Peptiden beschichteten Knochenzement-PMMA-Oberflächen:
x-Achse: RGD-Peptid Konzentration in der Beschichtungslösung (µM),
y-Achse: Zellbelegungsgrad (%)
*obere Kurve:* cyclo-RGDfK NH-CO - Acrylat-Derivat (Typ: iiia, Acrylat-peptid 3),
*mittlere obere Kurve*: cyclo-RGDfK NH-CO - Acrylat-Derivat (Typ: iib, Acrylatpeptid 2),
*mittlere untere Kurve*: cyclo-RGDfK NH-CO - Acrylat-Derivat (Typ:iiib, Acrylatpeptid 4),
*untere Kurve*: cyclo-RGDfK NH-CO - Acrylat-Derivat (Typ: iia, Acrylat-peptid 1),

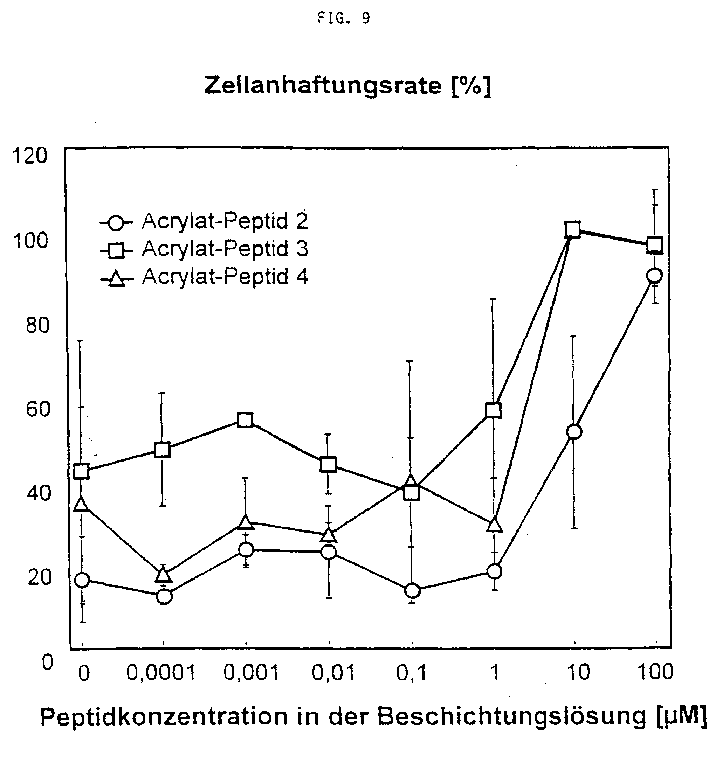

- Abb.9:: Adhäsion von MC3T3 H1-Osteoblasten an mit verschiedenen RGD-Peptiden beschichteten, porösen PMMA / PHEMA-Oberflächen:
x-Achse: RGD-Peptid Konzentration in der Beschichtungslösung (µM),
y-Achse: Zellbelegungsgrad (%)
*obere Kurve:* cyclo-RGDfK NH-CO - Acrylat-Derivat (Typ: iiia, Acrylat-peptid 3),
*mittlere Kurve*: cyclo-RGDfK NH-CO - Acrylat-Derivat (Typ: iiib, Acrylatpeptid 4),
*untere Kurve*: cyclo-RGDfK NH-CO - Acrylat-Derivat (Typ: iib, Acrylat-peptid 2),

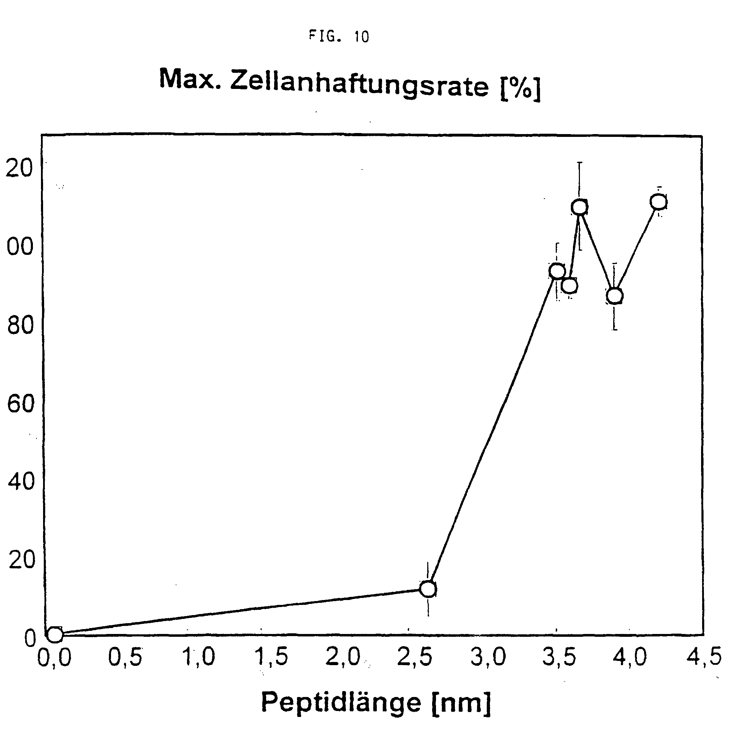

- Abb. 10:: Adhäsion von MC3T3 H1-Osteoblasten an mit verschiedenen RGD-Peptiden beschichteten, porösen PMMA / Plex Y7H-Oberflächen:
x-Achse: RGD-Peptid Konzentration in der Beschichtungslösung (µM),
y-Achse: Zellbelegungsgrad (%)
*obere Kurve:* cyclo-RGDfK NH-CO - Acrylat-Derivat (Typ: iiia, Acrylat-peptid 3),
*mittlere Kurve*: cyclo-RGDfK NH-CO - Acrylat-Derivat (Typ: iiib, Acrylatpeptid 4),
*untere Kurve*: cyclo-RGDfK NH-CO - Acrylat-Derivat (Typ: iib, Acrylat-peptid 2),

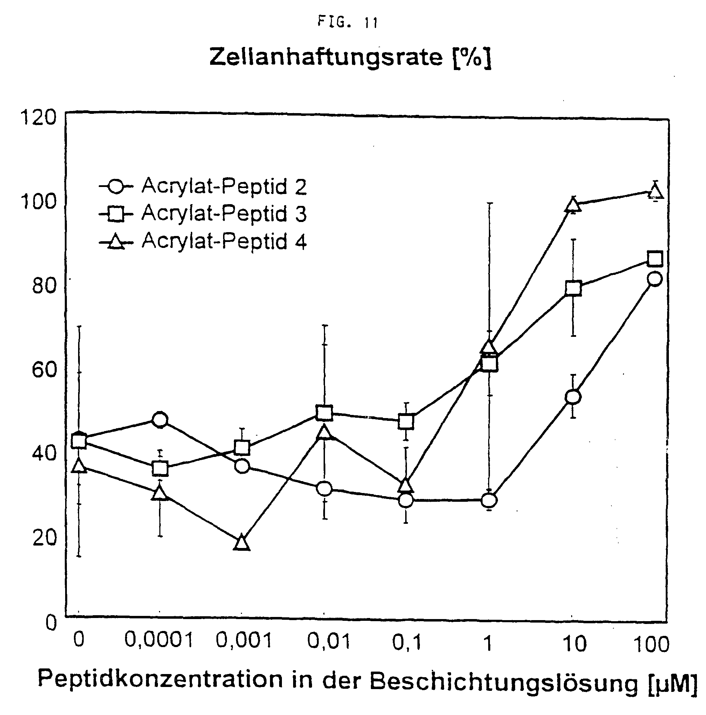

- Abb.11:: Maximale Adhäsion von MC3T3 H1-Osteoblasten an mit verschiedenen RGD-Peptiden verschiedener Moleküllängen beschichteten Knochenzement-PMMA- bzw. Polystyrol-BSA-SMPB-Oberflächen:
x-Achse: RGD-Peptid-Moleküllängen (nm)
y-Achse: maximaler Belegungsgrad (%).

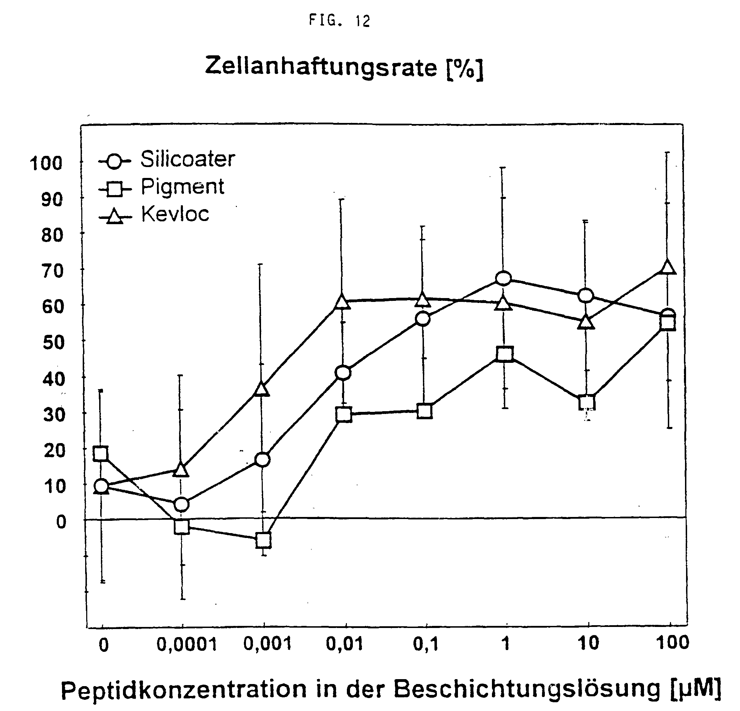

- Abb. 12:: Adhäsion von MC3T3 H1-Osteoblasten an mit RGD-Peptid cyclo-RGDfK NH-CO-Acrylat-Derivat (Typ iiia, Acrylatpeptid 3)- beschichteten V4A-Edelstahloberflächen. Die Edelstahloberflächen sind ihrerseits zuvor mit verschiedenen Verfahren beschichtet worden.
x-Achse: RGD-Peptid-Konzentration in der Beschichtungslösung (µM)
y-Achse: Zellbelegungsgrad (%);
*obere Kurve*: Kevloc®-Beschichtung;
*mittlere Kurve*: Silicoater®-Beschichtung;
*untere Kurve*: Pigmentbeschichtung.

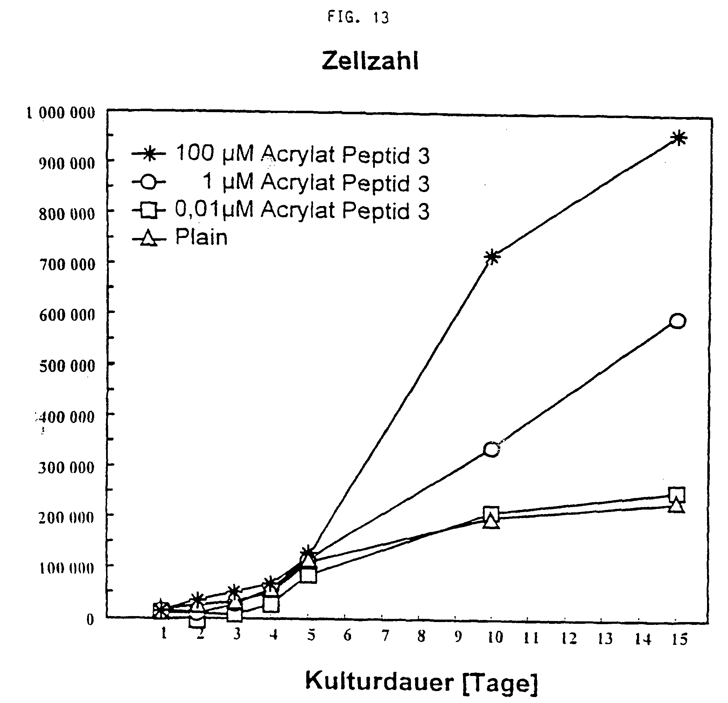

- Abb. 13:: Proliferation von MC3T3 H1-Osteoblasten an mit RGD-Peptid cyclo-RGDfK NH-CO-Acrylat-Derivat (Typ iiia, Acrylatpeptid 3)- beschichteten Knochenzement-PMMA-Oberflächen.
x-Achse: Kultivierungsdauer (Tage),
y-Achse: Zellzahl,
*oberer Kurve*: 100 µM Peptid in der Beschichtungslösung;
*mittlere obere Kurve*: 1 µM Peptid in der Beschichtungslösung;
*mittlere untere Kurve*: 0,1 µM Peptid in der Beschichtungslösung;
*untere Kurve*: unbeschichtete Kontrolle.

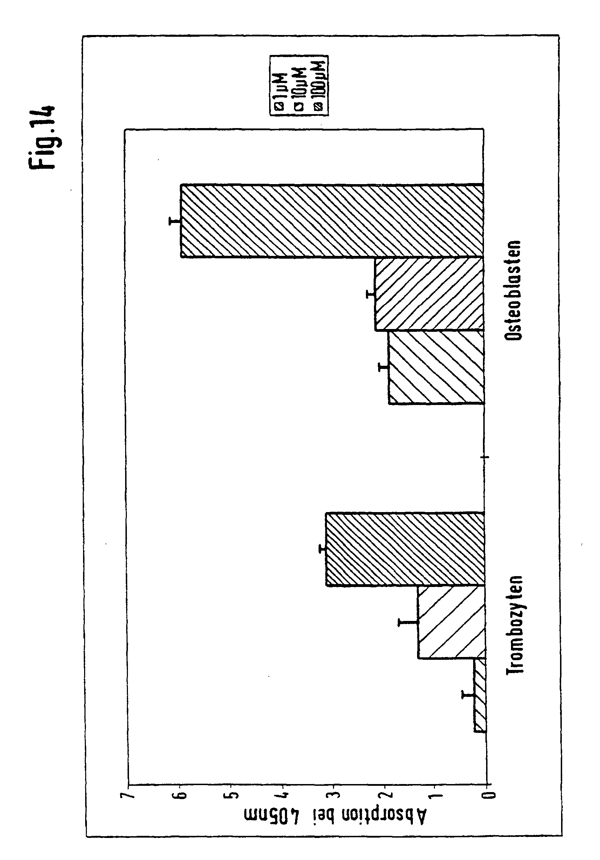

- Abb. 14:: Adhäsion von MC3T3 H1-Osteoblasten und Thrombozyten an mit RGD-Peptid cyclo RGDfK NH-CO-Acrylat-Derivat (Typ: iiia, -Acrylatpeptid 3) beschichteten Knochenzement-PMMA-Oberflächen.
x-Achse: Osteoblasten alleine und Thrombozyten alleine angesät auf Oberflächen mit verschiedenen Peptidkonzentrationen in der Beschichtungslösung von 100 µM, 10 µM und 1 µM.
y-Achse: Zellzahl (Absorption bei 405 nm).

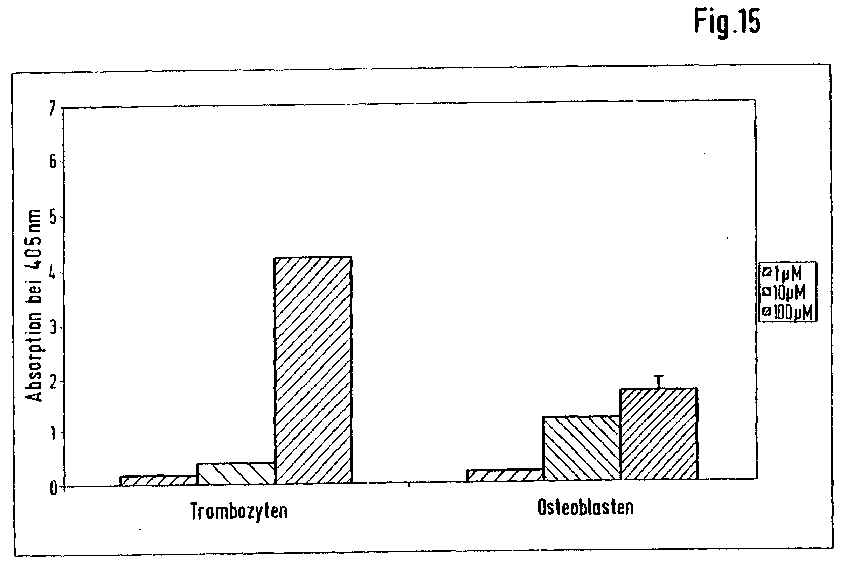

- Abb. 15:: Adhäsion von MC3T3 H1-Osteoblasten und Thrombozyten an mit RGD-Peptid cyclo RGDfKG NH-CO-Acrylat-Derivat (Typ: iiia, Acrylatpeptid 5) beschichteten Knochenzement-PMMA-Oberflächen.
x-Achse: Osteoblasten alleine und Thrombozyten alleine angesät auf Oberflächen mit verschiedenen Peptidkonzentrationen in der Beschichtungslösung von 100 µM, 10 µM und 1 µM.
y-Achse: Zellzahl (Absorption bei 405 nm).

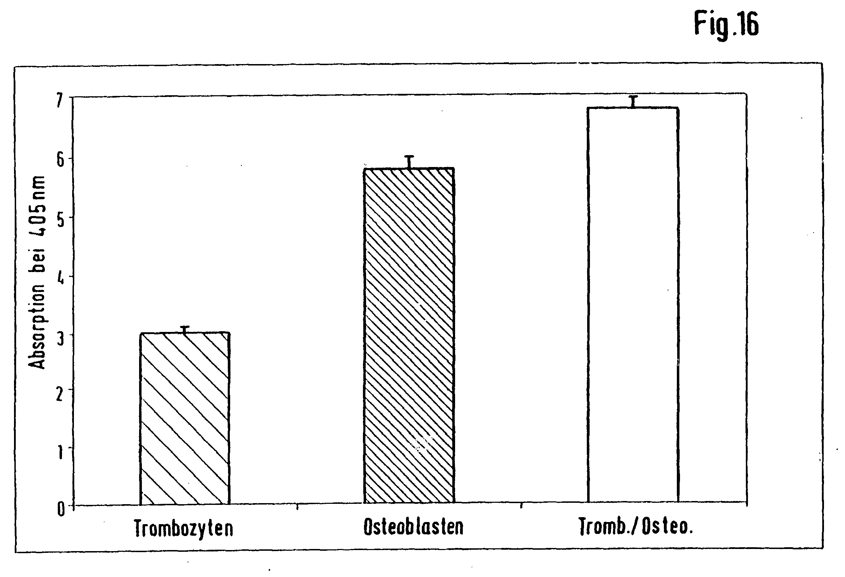

- Abb. 16:: Adhäsion von MC3T3 H1-Osteoblasten, Thrombozyten und einem Osteoblasten/Thrombozyten-Gemisch an mit RGD-Peptid cyclo RGDfK NH-CO-Acrylat-Derivat (Typ: iiia, Acrylatpeptid 3) beschichteten Knochenzement-PMMA-Oberflächen.
x-Achse: Osteoblasten alleine, Thrombozyten alleine undOsteoblasten / Thrombozyten-Gemisch angesät auf Oberflächen mit der
Peptidkonzentration in der Beschichtungslösung von 100 µM. y-Achse: Zellzahl (Absorption bei 405 nm)

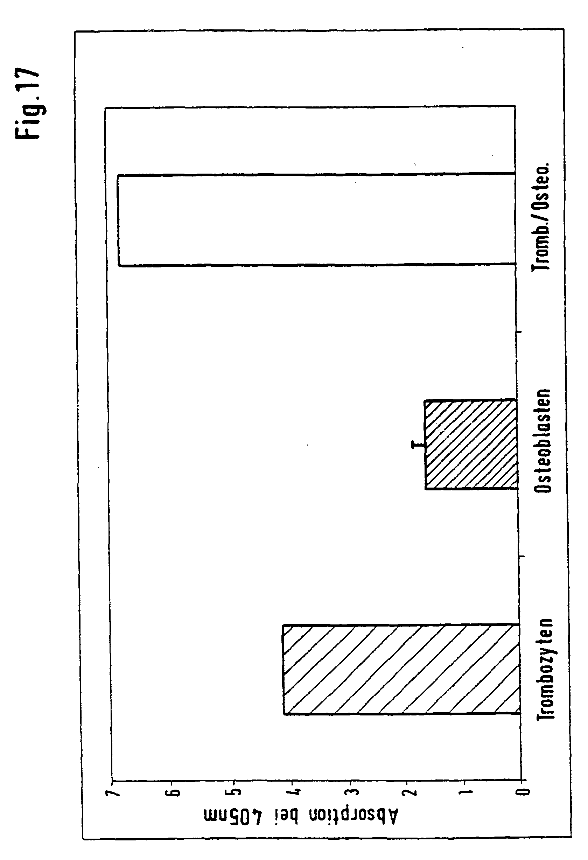

- Abb. 17:: Adhäsion von MC3T3 H1-Osteoblasten, Thrombozyten und einem Osteoblasten/Thrombozyten-Gemisch an mit RGD-Peptid cyclo RGDfKG NH-CO-Acrylat-Derivat (Typ: iiia, Acrylatpeptid 5) beschichteten Knochenzement-PMMA-Oberflächen.
x-Achse: Osteoblasten alleine, Thrombozyten alleine und Osteoblasten /Thrombozyten-Gemisch angesät auf Oberflächen mit der Peptidkonzentration in der Beschichtungslösung von 100 µM.
y-Achse: Zellzahl (Absorption bei 405 nm).

Die folgenden Beispiele erläutern weiter die Erfindung, ohne sie dabei zu beschränken.

### Beispiel 1:

(a) Die Synthese des cyclo-RGD-Peptids (Arg-Gly-Asp-DPhe-Lys) mit dem Anker Mercaptopropionsäure (→ cyclo-RGDfK NH-CO - Thiol-Derivat Typ: ia) wurde entsprechend dem Verfahren aus der DE 1 95 38 741 durchgeführt. Analog wurde die Synthese des in seiner biologischen Wirkung inaktiven entsprechenden cyclo-RβADfK-Derivats sowie des cyclo-RGDfK-Derivats ohne Ankermolekül durchgerührt.
(b) cyclo (R(Pbf)GD(tBu)fK(Z)) (Pbf = pentamethyl-benzofuran-sulfonyl; tBu = tert. Butyl) wurde durch Festphasenpeptidsynthese (Merrifield, Angew. Chem. 1985, 97: 801) und anschließender Cyclisierung ( z. B. nach Zimmer et al., 1993, Liebigs Ann. Chem: 497) erhalten. Nach selektiver Abspaltung der Z-Schutzgruppe nach Standardmethodik kann die Lysinseitenkette durch Umsetzen von 0.1 mmol cyclo (R(Pbf)GD(tBu)fK) mit 0.2 mmol Bernsteinsäureanhydrid (bsa) in 5 ml Dimethylformamid zu cyclo (R(Pbf)GD(tBu)f[bsa-K]) verlängert werden.
(c) Die Synthese des cyclo-RGD-Peptids (Arg-Gly-Asp-DPhe-Lys) mit dem Anker Mercaptoethylamidobernsteinsäure (→ cyclo-RGDfK NH-CO - Thiol-Derivat Typ: ib = cyclo(RGDf[Thiol-bsa-K]) wurde wie folgt, durchgeführt: 10 mmol Cysteaminhydrochlorid und eine äquimolare Menge (eq.) an Triphenylmethanol wurden bei 60°C in Eisessig gelöst und unter Rühren mit 1.1 eq. BF₃-Etherat versetzt. Nach 50 minütigem Rühren wurde mit gesättigter NaHCO₃-Lösung neutralisiert und mit Essigester extrahiert. Das aus dem Extrakt verbleibende Öl wurde zur Überführung in das Hydrochlorid in tert.-Butanol gelöst, mit verdünnter Salzsäure auf pH = 2 gebracht und gefriergetrocknet. Ausbeute 99%. Der so erhaltene Baustein wird mit EDCl xHCl nach Standardmethoden an cyclo (R(Pbf)GD(tBu)f[bsa-K]) gekuppelt und die Seitenschutzgruppen werden abgespalten.
(d) Die Synthese des cyclo-RGD-Peptids (Arg-Gly-Asp-DPhe-Lys) mit dem Anker Acrylamidohexansäure (→ cyclo-RGDfK NH-CO-Acryl-Derivat Typ: iia = cyclo(RGDf[Acryl-ahx-K]) wurde wie folgt, durchgeführt:
   Das Acrylankersystem wurde seperat synthetisiert und als Aktivester an die Peptidseitenkette gekuppelt. Dazu wurden 10 mmol 6-Aminohexansäure und 1.8 eq. Calciumhydroxid in Wasser suspendiert und bei 0° C 1,2 eq. Acrylsäurechlorid zugegeben. Unlösliches Calciumhydroxid wurde abfiltriert und das Filtrat mit konzentrierter Salzsäure auf pH 2 angesäuert. Das ausgefallene Produkt wurde aus Essigester rekristallisiert. Ausbeute: 65%. 10 mmol der kristallinen 6-Acrylamidohexansäure wurden in 50 ml Dichlormethan suspendiert, mit 1 eq. N-Hydroxysuccinimid versetzt und bei 0°C 1.2 eq. EDClxHCl zugegeben. Nach einstündigem Rühren wurde die Reaktion durch Zusatz von 10 µl Eisessig gestoppt. Es wurde mehrfach mit kalter, gesättigter Natriumcarbonatlösung und mit Wasser extrahiert und anschließend über Natriumsulfat getrocknet. Ausbeute: 52%. Der nun vorliegende Aktivester wurde in DMF an cyclo(R(Pbf)GD(tBu)fK) angekuppelt und die Seitenkettenschutzgruppen nach Standardvorschriften abgespalten.
(e) Die Synthese des cyclo-RGD-Peptids (Arg-Gly-Asp-DPhe-Lys) mit dem Anker Acrylamidohexansäure-amidohexansäure (→ cyclo-RGDfK NH-CO-Acryl-Derivat Typ: iib = cyclo(RGDf[Acryl-ahx-ahx-K]) wurde wie folgt, durchgeführt: Zur Synthese des Acrylankersystems werden 10 mmol Aminohexansäure in wäßrigem Natriumphosphatpuffer (pH 8) gelöst und auf 0°C gekühlt. 2 mmol Acrylamidohexansäureaktivester (siehe (d)) wurden in Ethanol/CHCl₃ gelöst und langsam zugegeben. Der pH-Wert wurde mit verdünnter NaOH konstant auf 8 gehalten. Nach Abdestillieren der organischen Lösungsmittel wurde die wäßrige Phase mit konzentrierter Salzsäure auf pH 2.6 angesäuert und der ausfallende Feststoff abfiltriert, mit Wasser gewaschen und über Phosphorpentoxid getrocknet. Ausbeute: 70%. Das nun vorliegende Acrylankersystem wird mit EDClxHCl nach an sich bekannten Methoden an die Peptidseitenkette gekuppelt und die Schutzgruppen abgespalten.
(f) Die Synthese des cyclo-RGD-Peptids (Arg-Gly-Asp-DPhe-Lys) mit dem Anker Acrylamidohexansäure-amido-triethylenglycolsäure (→ cyclo-RGDfK NH-CO-Acryl-Derivat Typ: iiia = cyclo(RGDf[Acryl-ahx-tEG-K]) wurde wie folgt, durchgeführt: Das Acrylankersystem wurde durch Festphasenpeptidsynthese (siehe oben) dargestellt. Als letzter Baustein wurde die Acrylamidohexansäure (siehe oben) gekuppelt. Ausbeute: 97 %. Das nun vorliegende Acrylankersystem wird mit EDClxHCl nach an sich bekannten Methoden an die Peptidseitenkette gekuppelt und die Schutzgruppen abgespalten.
(g) Die Synthese des cyclo-RGD-Peptids (Arg-Gly-Asp-DPhe-Lys) mit dem Anker Acrylamidohexansäure-amidotriethylenglycolsäure-amidotriethylen-glycolsäure (→ cyclo-RGDfK NH-CO-Acryl-Derivat Typ: iiib = cyclo (RGDf [Acryl-ahxtEG-tEGK]) erfolgte analog zu (f). Ausbeute: 85%.
(i) Die Synthese des cyclo-RGD-Peptids (Arg-Gly-Asp-DPhe-Lys-Gly) mit dem Anker Acrylamidohexansäure-amidotriethylenglycolsäure (→ cyclo-RGDfK NH-CO-Acryl-Derivat Typ: iiia = cyclo (RGDf [Acryl-ahx-tEG-KG]) erfolgte analog zu (f). Ausbeute: 81 %.

### Beispiel 2:

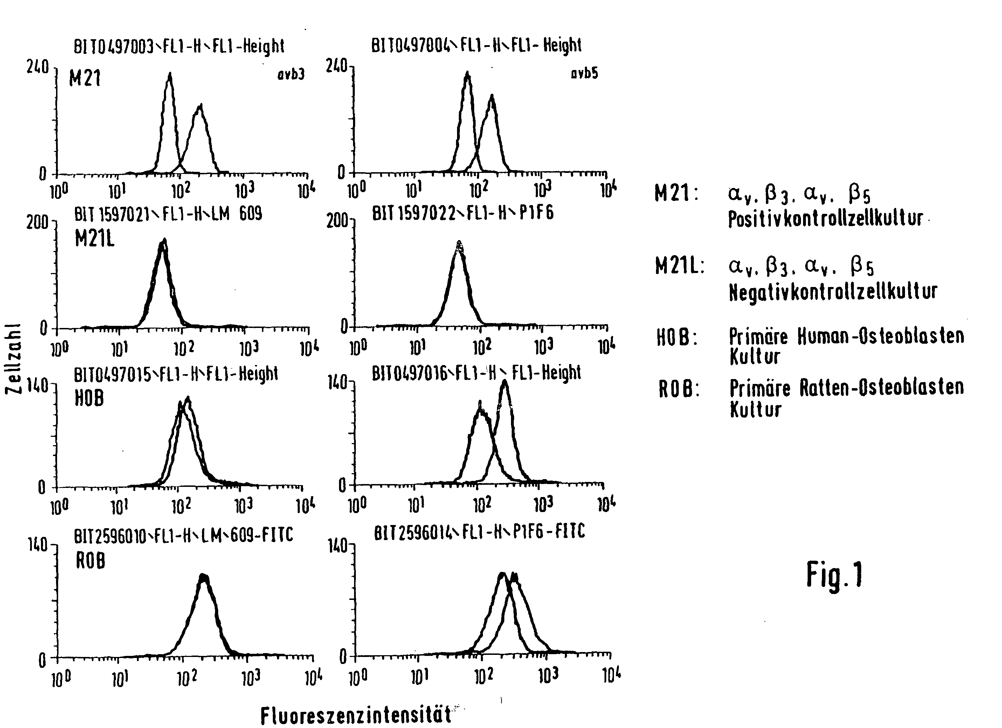

In Anlehnung an die etablierten Verfahren von Singer et al., 1987, J. Cell. Biol. 104:573, bzw. von Ruoslahti et al. (1982, Methods. Enzymol., 32:803-831) wurde die kovalente Kopplung der erfindungsgemäßen Peptide mit Sulfo-Succinimidyl-4 (p-Maleimidophenyl)-Butyrat (Sulfo-SMPB) an mit Rinderserumalbumin (BSA) vorbeschichteten Kulturoberflächen aus Polystyrol vollzogen.
Hierzu wurden 48-Wells (Costar, "non-tissue culture treated", Art. Nr. 3547) mit je 250µl PBS (phosphate buffered saline), pH = 8,3, 2% BSA, pro Vertiefung überschichtet und über Nacht bei Raumtemperatur inkubiert, um eine auf dem Polystyrol befindliche BSA-Schicht zu generieren. Anschließend wurde mit 250 µl PBS, pH = 8,3 pro Vertiefung gewaschen und mit je 250 µl einer Lösung mit 100 µg/ml SMPB in PBS, pH 8,3, l Stunde bei Raumtemperatur inkubiert. Danach erfolgte ein dreimaliges Waschen der Wells mit je 250 µl PBS, pH 8,3. Zur Herstellung der Beschichtungslösungen wurde das entsprechende Thiol-RGD-Peptid in folgenden Endkonzentrationen in PBS, pH 8,3, angesetzt: 1 nM, 10 nM, 100 nM, 1 µM, 10 µM, 100 µM, und 1 mM. Nach Zugabe von je 250 µl Beschichtungslösung/Well wurde über Nacht bei Raumtemperatur inkubiert und anschließend dreimal mit PBS, pH 8,3, gewaschen. Durch Zugabe von je 250 µl einer 5%igen BSA-Lösung in PBS, pH 7,4, anschließender Inkubation für 2 Stunden bei Raumtemperatur und Waschen mit PBS, pH 7,4, wurden unspezifische Zellbindestellen blockiert. Als Beschichtungs-Negativ-Kontrollen fungierten Polystyroloberflächen, die ab der Beschichtung mit einer 5%igen BSA-Lösung identisch wie die Positivproben behandelt wurden. Anschließend wurde die Adhäsion von vier Zellkulturen, die aus drei unterschiedlichen Spezies gewonnen wurden, an die oben genannten mit RGD-Peptid beschichteten Oberflächen untersucht:
- primäre humane Osteoblasten aus der Femurkopfspongiosa adulter Patienten (Siggelkow et al., 1997, Bone 20: P231);
- primäre humane Osteoprogenitorzellen aus dem Knochenmark adulter Patienten (Vilamitjana-Amedee et al., 1993, In vitro Cell Dev. Biol. 29: 699);
- primäre Osteoblasten aus der Kalvaria neonataler Ratten, deren Präparation und Kultivierung in Anlehnung an die Methode von Yagiela und Woodbury (1977, The Anatomical Record, 188:287-305) erfolgte;
- Osteoblasten-Zellinie MC3T3 H1, gewonnen aus der Kalvaria neonataler Mäuse (Heermeier et al., 1995, Cells and Materials, 5:309-321) und
- Humane Melanom-Zell-Linie M21L als alphaᵥbeta₃/alphaᵥβ₅-Integrin Negativkontrolle.
Bevor diese Zell-Spezies zu den Adhäsionsexperimenten eingesetzt wurden, wurde deren Integrin-Expression mittels fluoreszenzmarkierter Antikörper gegen die alphaᵥbeta₃- bzw. alphaᵥbeta₅-Rezeptoren sowie gegen die Integrin-Untereinheiten alphaᵥ, beta₃ und beta₅ anhand eines Becton-Dickenson Fluorescence Activated Cell Sorter (FACS) verifiziert. Es zeigte sich dabei eine starke Expression der besagten Integrine (Abb. 1).
Die beschriebenen Zell-Spezies wurden mit einer Ansaatdichte von 48.000 Zellen/cm² in, wie oben beschrieben, mit RGD-Peptid beschichteteten Costar-48-Wells, angesät und anschließend 1 Stunde bei 37°C, 95% Luftfeuchte und 5% CO₂ inkubiert. Anschließend wurden während des Experiments nicht adherierte Zellen zweimal mit PBS, pH 7,4, abgewaschen.
Die Quantifizierung der adherierten Zellen erfolgte indirekt über die Aktivitätsbestimmung des zellulären Enzyms N-Acetyl-Hexosaminidase unter Verwendung einer entsprechenden Standardkurve nach der Methode von Landegren (1984, J. Immunol. Methods, 67:379-388).
Die Beschichtung von BSA-vorbehandelten Polystyrol-Zellkulturoberflächen mit den alphaᵥbeta₃- bzw. alphaᵥbeta₅-selektiven Thiolpeptiden 1-, 2- (Sulfo-SMPB)-Derivaten führt zu einer starken und dosisabhängigen Stimulierung der Adhäsion von kultivierten Maus MC3T3 H1-Osteoblasten (s. Abb. 2). Im Gegensatz hierzu hat die biologisch inaktive Thiolpeptid-Kontrolle keinen signifikanten Effekt, wodurch die hohe Selektivität und die hohe Spezifität der Integrin-RGD-Peptid Wechselwirkung gezeigt wird. Auch das weniger alphaᵥbeta₃-/ alphaᵥbeta₅-selektive Thiolpeptid 3 -(Sulfo-SMPB) (cyclo-RGDvE-aminocystein-amidohexansäure: Delforge et al., 1996, Anal. Biochem. 242, 180) zeigt einen deutlich schwächeren Effekt als die entsprechenden Thiolpeptid 1 und 2 - derivate.
Das Bindungsverhalten der unterschiedlichen Zell-Spezies an mit Thiolpeptid 1-(Sulfo-SMPB)-Derivat oder Thiolpeptid 2-(Sulfo-SMPB)-Derivat beschichtete, BSA-vorbehandelte Polystyrol-Oberflächen entspricht einer Titrationskurve mit einer maximalen Adsorptionsrate von ca. 70% (primäre Ratten-Osteoblasten), ca. 80% (primäre humane Osteoblasten und primäre humane Osteoprogenitorzellen) und ca. 90-100% (MC3T3 H1- Maus-Osteoblasten) der angesäten Zellen sowie einer halbmaximalen Zellanbindung bei einer RGD-Peptid-Konzentration in der Beschichtungslösung von 50 - 1000 nM (Abb.3). Alpha ᵥbeta₃- / alphaᵥbeta₅- Negativkontrollzellen (M21L) zeigen im Gegensatz hierzu keine signifikante Zell-Adhäsion
Die beschriebenen Zell-Adsorptionsraten sind gegenüber mit 5% BSA-beschichteten Oberflächen, welche zu keiner signifikanten Zell-Adhäsion für alle eingesetzten Osteoblasten Kulturen führen, ca. 20-40 fach erhöht.
Das ähnliche Verhalten der genannten Osteoblasten-Kulturen belegt, daß die adhäsionsstimulierenden Effekte osteoblastenspezifisch jedoch speziesunabhängig sind.

Die Bindung der oben genannten Osteoblasten-Zellkulturen an mit Thiolpeptid 1-Sulfo-SMPB-Derivat beschichteten BSA-vorbehandelten Polystyroloberflächen kann durch Zugabe von gelöstem zyklischen RGDfK im Anhaftungsmedium vollständig inhibiert werden (Abb. 4). Dieses Ergebnis beweist, daß die beobachteten Zelladhäsionsphänomene alleine durch RGD-Peptide vermittelt werden.
Die Abhängigkeit der Zelladhäsion von primären humanen Osteoblasten bzw. von MC3T3 H1-Zellen auf den genannten Thiolpeptid 1,2 (Sulfo-SMPB) beschichteten, BSA-vorbehandelten Polystyroloberflächen von der Anzahl angesäter Zellen liefert eine Sättigungskurve (Abb. 5). Auf der BSA-Negativbeschichtung dagegen ist keine signifikante Zellanhaftung zu erkennen. Die bei maximaler Zellaussaatdichte berechnete durchschnittliche Fläche pro adherierte Zelle von ca. 200 bis 500 µm² liefert auf dieser Oberfläche einen durchschnittlichen Zell-Zell-Abstand von etwa 15 bis 25 µm (Abb. 6 ). Da der Osteoblasten-Zelldurchmesser ca. 10 bis 15 µm beträgt, wird deutlich, daß die Peptidbeschichtung Zellanhaftungsstellen in solcher Zahl bereitstellt, daß eine mit Osteoblasten dicht belegte Oberfläche erzielt werden kann, bei der die Zellen in unmittelbarer Nachbarschaft zueinander angeordnet sind. Der zeitliche Verlauf des Zeilanhaftungsprozesses auf der genannten Oberfläche zeigt eine schnelle Adhäsion der Zellen, die, je nach Zelltyp, nach etwa 45 bis 60 Minuten abgeschlossen ist (Abb. 7 ). Zell-Linien (z.B. MC3T3 H1) zeigen dabei eine schnellere Anhaftungskinetik als primäre Zellkulturen (Human, Ratte). Signifikante Unterschiede zwischen den beiden verwendeten Thiolpeptid-Derivaten sind bei diesen Versuchen nicht erkennbar.

### Beispiel 3:

Zur Untersuchung des Einflusses verschiedener Ankermoleküllängen und damit des Abstandes zwischen der zellerkennenden RGD-Sequenz und der Materialoberfläche auf das Ausmaß der Osteoblasten-Adhäsion wurden vier verschiedene Acrylat-RGD-Peptide (cyclo-RGDfK NH-CO - Acrylat-Derivat (Typ: iia, iib, iiia, iiib), mit unterschiedlichen molekularen Abstandshalterlängen hergestellt.

Die Synthesen der cyclo-RGD-Peptide wurden, wie in Beispiel 1 angegeben oder entsprechend den Verfahren von Pless et al., 1983, J. Biol. Chem. 258: 2340-2349 bzw. von Gurrath et al., 1992, Eur. J. Biochem. 210: 911-921 durchgeführt.
Die entsprechenden Ankerlängen dieser Peptide betragen etwa 2,6 nm (Acrylatpeptid 1, Typ iia), 3,5 nm (Acrylatpeptid 2, Typ iib), 3,7 nm (Acrylatpeptid 3, Typ iiia) bzw. 4,2 nm (Acrylatpeptid 4, Typ iiib).

Zur kovalenten Kopplung dieser Peptide an auspolymerisierte PMMA (Polymethylmethacrylat) -Oberflächen wurden entsprechende Formkörper unterschiedlicher Porosität (Durchmesser: 10 mm; Höhe: 2 mm) aus drei verschiedenen PMMA-Komponenten hergestellt.
- Knochenzement auf PMMA-Basis (Fa. Merck KGaA, Germany, Zul. Nr. 5181.00.00) entsprechend der Produktbeschreibung (40 g Pulver + 20 ml Flüssigkeit).
- 10 g PMMA/PHEMA (Polyhydroxyethylmethacrylat)-Granulat, Partikelgröße 0,5-0,6 mm (Fa. Biomet) wurden mit 1,3 ml einer HEMA-Lösung (68,6% HEMA, 29,4% TEGMA, [Triethylenglykoldimethacrylat], 2% tBPB, [tert. Butylperoxybenzoat]) vermischt und dadurch zu einem porösen Trägermaterial verklebt.
- 10 g PMMA-Granulat (Plex Y7H, Röhm, Germany), Kornfraktion 0,7-2 mm wurden mit 1,5 ml einer Methylmethacrylat (MMA)-Lösung (68,6% MMA, 29,4% TEGMA 2% tBPB) vermischt und dadurch zu einem porösen Trägermaterial verklebt.

Zur kovalenten Kopplung der Acrylatpeptide an die vorpolymerisierten PMMA-Formkörper wurden Stammlösungen der Acrylatpeptide 1, 2, 3 und 4 in einer Endkonzentration von jeweils 100 µM in DMSO/0,2% Campherchinon (w/v) hergestellt. Anschließend wurden durch Verdünnung mit Isopropanol/0,2% Campherchinon (w/v) Konzentrationsreihen mit Peptidendkonzentrationen von jeweils 0,1 nM, 1 nM, 10 nM, 100 nM, 1 µM und 10 µM hergestellt. Die Formkörper wurden 2 Stunden bei Tageslicht und Raumtemperatur inkubiert und anschließend in trockenem Zustand bei 4°C gelagert. Vor Gebrauch wurden die Proben zur Entfernung ungebundener Peptide dreimal mit PBS, pH 7,4, gewaschen und über Nacht in PBS, pH 7,4, bei 4°C gelagert.
Durch Zugabe von je 250 µl/Formkörper einer 5%igen BSA-Lösung in PBS, pH 7,4, und anschließender Inkubation für 2 Stunden bei Raumtemperatur und einmaligem Waschen mit PBS, pH 7,4, wurden unspezifische Zellbindestellen blockiert.
Als Negativkontrollen fungieren PMMA-Formkörper, die anstelle mit den Peptidlösungen mit einer Lösung aus Isopropanol/0,2% Campherchinon (w/v) behandelt wurden.

Im Anschluß wurde die Adhäsion von Osteoblasten der MC3T3 H1-Linie, gewonnen aus der Kalvaria neonataler Mäuse (Heermeier et al., 1995, Cells and Materials 5: 309-321) an o. b. peptidbeschichtete Oberflächen untersucht.
Die MC3T3 H1-Osteoblasten wurden mit einer Ansaatdichte von 48.000 Zellen/cm² auf, wie oben beschrieben, in Costar-48-Wells befindlichen, mit RGD-Peptid beschichteten, PMMA-Formkörpern angesät und anschließend 1 Stunde bei 37°C, 95% Luftfeuchte und 5% CO₂ inkubiert. Anschließend wurden während des Experiments nicht adherierte Zellen zweimal mit PBS, pH 7,4, abgewaschen.
Die Quantifizierung der adherierten Zellen erfolgte indirekt über die Aktivitätsbestimmung des zellulären Enzyms N-Acetyl-Hexosaminidase unter Verwendung einer entsprechenden Standardkurve nach der Methode von Landegren (1984, J. Immunol. Methods, 67: 379-388).

Das Bindungsverhalten der MC3T3 H1-Osteoblasten an mit genannten Acrylat-RGD-Peptiden 1 (Typ iia), 2 (Typ iib), 3 (Typ iiia) und 4 (Typ iiib) beschichteten, unterschiedlichen PMMA-Formkörpern entspricht jeweils einer Titrationskurve. Die maximalen Adsorptionsraten betragen ca. 20% für Acrylatpeptid 1 und ca. 80-100% für die Acrylatpeptide 2, 3 und 4. Die halbmaximalen Zellanbindungen erfolgen bei einer RGD-Peptidkonzentration in der Beschichtungslösung von ca. 1-10000 nM für die Acrylatpeptide 2, 3 und 4.

Die beschriebenen Zell-Adsorptionsraten auf RGD-Peptid-beschichteten Knochenzement-Formkörpern sind gegenüber unbeschichteten Oberflächen ca. 1,5 fach (Acrylatpeptid 1) und ca. 5-15 fach (Acrylatpeptide 2, 3 und 4) erhöht (Abb. 8).
Die beschriebenen Zell-Adsorptionsraten auf RGD-Peptid-beschichteten porösen PMMA/PHEMA-Granulat-Formkörpern sind gegenüber unbeschichteten Oberflächen 2-5 fach (Acrylatpeptide 2, 3 und 4) erhöht (Abb. 9).
Die beschriebenen Zell-Adsorptionsraten auf RGD-Peptid-beschichteten porösen Plex Y7H-Granulat-Formkörpern sind gegenüber unbeschichteten Oberflächen 2-3 fach (Acrylatpeptide 2, 3 und 4) erhöht (Abb. 10).
Für PMMA/PHEMA- bzw. für PMMA-Plex Y7H-Proben werden sowohl eine größere Schwankungsbreite der Zelladhäsionswerte als auch eine stärkere Anhaftung der Osteoblasten an unbeschichtete Kontrollproben bei gleichbleibenden maximalen Anhaftungsraten an RGD-Peptid-beschichtete Proben beobachtet als für PMMA-Knochenzementformkörper.

Für die Acrylatpeptide 2, 3 und 4 werden sowohl im Vergleich untereinander als auch im Vergleich zu den auf der Test-BSA-Kontrolle beschichteten Thiolpeptiden 1 und 2 sehr ähnliche Stimulierungen der Zell-Adhäsion beobachtet, das kürzeste Peptid, Acrylatpeptid 1, ist dagegen fast inaktiv. Daraus kann geschlossen werden, daß ein kritischer Mindestabstand zwischen RGD-Zellerkennungssequenz und der Materialoberfläche von ca. 2,5-3,5 nm erforderlich ist, um eine sterisch erfolgreiche Zell-Adhäsion vollziehen zu können (Abb. 11).
Die höhere Aktivität von Acrylatpeptid 3 auf dem Knochenzement-PMMA-Träger weist auf eine für die Zellanhaftung optimale Molekülstruktur hinsichtlich sowohl der Moleküllänge als ggf. auch der Hydrophilie/Hydrophobie-Verteilung bzw. -Verhältnis im Abstandshalter hin.

### Beispiel 4:

Zur Testung der Zelladhäsion von Osteoblasten an peptidbeschichtete Metalloberflächen wurden V4A-Edelstahlformkörper (7x7x1 mm³) mit drei verschiedenen Beschichtungsvarianten versehen und jeweils anschließend ihrerseits mit RGD-Peptiden beschichtet. Dazu wurden vorerst die Edelstahlformkörper zwecks Reinigung 15 min. bei 60°C im Ultraschallbad inkubiert, mit VE-Wasser gespült, 30 min. in Aceton inkubiert, nochmals mit VE-Wasser gespült und zur Trocknung 24 Stunden bei 60°C inkubiert.

Anschließend wurden die Edelstahlplättchen mittels dreier Varianten beschichtet:
*a. Kevloc®-Verfahren* (Fa. Heraeus Kulzer GmbH, Wehrheim, Germany):
   Die Lösung Kevloc®-Primer wurde dünn aufgebracht und 3 min. bei Raumtemperatur inkubiert. Anschließend wurde die Lösung Kevloc®-Bond ebenfalls dünn aufgebraucht und 20 min. bei 180°C im Brennofen aktiviert.
   Da diese Beschichtungsvariante direkt freie Acrylatreste auf der Metalloberfläche bereitstellt, konnte anschließend das Acrylatpeptid 3, Typ iiia, direkt kovalent gekoppelt werden, so wie dies im Beispiel 3 für auspolymerisierten PMMA-Knochenzement beschrieben wurde.
*b. Silicoater®-Verfahren* (Fa. Heraeus Kulzer GmbH, Wehrheim, Germany):
   Die Lösung Siliseal® wurde dünn aufgebracht und 5 min. bei Raumtemperatur getrocknet. Anschließend wurde die Lösung Sililink ebenfalls dünn aufgebracht und 3 min. bei 320°C im Brennofen aktiviert.
   Zur Bereitstellung von Acrylatresten auf den beschichteten Metalloberflächen wurden die vorbehandelten Edelstahlformkörper in 75°C erwärmtes VE-Wasser, mit NaOH auf pH ca. 8,00 eingestellt und getaucht. Anschließend wurde über einen Zeitraum von einer Stunde eine 1%ige 3-Methacryloxypropyltrimethoxysilan-Lösung (Fa. Hüls AG, Germany) unter Rühren zudosiert, bis ein pH-Wert von ca. 6,50 erreicht wurde. Anschließend wurde eine Stunde bei 75°C nachgerührt, der pH-Wert lag zu diesem Zeitpunkt bei ca. 6,35.
   Die Anbindung von Acrylatpeptid 3, Typ iiia, erfolgte wie im Beispiel 3 für auspolymerisierten PMMA-Knochenzement beschrieben.
*c. Pigmentbeschichtung* (Fa. Merck KGaA, Darmstadt, Germany):
   Diese Beschichtungsvariante erfolgte entsprechend eines Silanisierungsverfahrens, das in der DE-OS 4321005 beschrieben ist, und welches ursprünglich die technische Lehre der Beschichtung von Perlglanzpigmenten für Wasserlacksysteme für Metalle und Kunststoffe in der Automobil- und Kunststoffindustrie darlegt.
   Hierzu wurden die Edelstahlformkörper in 75°C erwärmtes VE-Wasser, mit NaOH auf pH ca. 8,00 eingestellt, und es wurde über einen Zeitraum von zwei Stunden eine AlCl₃-Lösung unter Rühren zudosiert. Anschließend wurde eine 5%ige Natronwasserglas-Lösung ebenfalls unter Rühren über zwei Stunden zudosiert. Im folgenden wurde innerhalb einer Stunde eine 1%ige 3-Methacryloxypropyltrimethoxysilan-Lösung (Fa. Hüls AG, Germany) unter Rühren zudosiert. Anschließend wurde eine Stunde bei 75°C nachgerührt, der pH-Wert lag zu diesem Zeitpunkt bei ca. 6,40.
   Die Anbindung von Acrylatpeptid 3, Typ iiia, erfolgte wie im Beispiel 3 für auspolymerisierten PMMA-Knochenzement beschrieben.

Nachdem die Edelstahlformkörper mittels dreier verschiedener Verfahren mit Acrylatpeptid 3, Typ iiia, beschichtet worden waren, wurde die Adhäsion von Osteoblasten der Linie MC3T3 H1 untersucht. Hierzu wurde wie in Beispiel 3 beschrieben verfahren.
Das Bindungsverhalten der MC3T3 H1-Osteoblasten an mit genanntem Acrylatpeptid 3 beschichteten, mit drei unterschiedlichen Verfahren vorbehandelten V4A-Edelstahlformkörper entspricht jeweils einer Titrationskurve (Abb. 12).
Die maximalen Adhäsionsraten betragen ca. 60% für die Pigmentbeschichtung und ca. 80% für Kevloc®- bzw. Silicoater®-Verfahren. Die halbmaximalen Zellanbindungen erfolgen bei einer RGD-Peptidkonzentration in der Beschichtungslösung von ca. 1-100 nM für alle drei Beschichtungsvarianten. Die beschriebenen Zell-Adsorptionsraten auf RGD-Peptid-beschichteten Edelstahlformkörpem sind gegenüber unbeschichteten Kontrollen ca. 3-8 fach erhöht.
Für Kevloc®- bzw. Silicoater®-Verfahren wurden jeweils zusätzlich Beschichtungen unter Verwendung lediglich einer Beschichtungslösung hergestellt (Kevloc®-Primer alleine, Kevloc®-Bond alleine, Siliseal® alleine, Sililink® alleine). Es wurden dabei keine signifikanten Unterschiede in der Zelladhäsion im Vergleich zu den jeweiligen Doppelbeschichtungen (Kevloc®-Primer/-Bond, Siliseal®/Sililink®) beobachtet.

### Beispiel 5:

Zur ergänzenden Untersuchung nicht nur der Osteoblasten-Adhäsion, sondern auch deren Proliferation auf RGD-Peptid-beschichteten Oberflächen wurde wie folgt vorgegangen:
Zur kovalenten Kopplung von Acrylatpeptid 3, Typ iiia, an auspolymersierte PMMA-Knochenzementformkörper wurde ein Beschichtungsverfahren gewählt, das im Beispiel 3 beschrieben wurde.
Anschließend wurden MC3T3 H1-Maus-Osteoblasten wie ebenfalls im Beispiel 3 beschrieben an diese RGD-Peptid-beschichteten Knochenzementformkörper adheriert, allerdings mit zwei Unterschieden: es wurden 12.000 Zellen/cm² anstelle von 48.000 Zellen/cm² angesät und die Adhäsionszeit betrug zwei Stunden anstelle von einer Stunde. Außerdem wurden nur drei verschieden Peptidkonzentrationen in der Beschichtungslösung untersucht: 0,01 µM, 1 µM und 100 µM.
Nach dem Abwaschen nicht adhärenter Zellen wurden die MC3T3 H1-Osteoblasten in serumhaltigem (10%) Kulturmedium über einen Zeitraum von 15 Tagen bei 3.7°C unter 95% Luftfeuchte und 5% CO₂ kultiviert. Dabei wurde zweimal pro Woche das Medium gewechselt und nach 1, 2, 3, 4, 5, 10 und 15 Tagen die Zellzahl mittels des WST-1-Tests von Boehringer Mannheim, Germany, bestimmt. Hierbei zeigte sich jeweils ein typischer exponentieller Verlauf der Zellproliferation der kultivierten Osteoblasten (Abb. 13). Die maximal erreichte Zellzahl war direkt von der RGD-Peptidkonzentration in der Beschichtungslösung abhängig. So wurden nach 15 Kultivierungstagen pro cm² ca. 1.000.000 Zellen (100 µM Peptid), ca. 550.000 Zellen (1 µM Peptid), ca. 300.000 Zellen (0,01 µM Peptid) bzw. ca. 230.000 Zellen (Kontrolle ohne Peptid) erreicht. Bei der höchsten Peptidkonzentration von 100 µM wurde somit eine etwa 4-5 fache Steigerung der Zellproliferation im Vergleich zu unbeschichteten Proben beobachtet.

### Beispiel 6:

Als Modell für die Materialbeschichtung mit RGD-Peptiden unterschiedlicher Zellselektivität zur Gewinnung von Implantaten mit lokal differenzierten bioaktiven Beschichtungsmustem, welche die Adhäsion verschiedener Zell-Species stimulieren sollen, dienten auspolymerisierte PMMA-Knochenzementformkörper. An diese wurden zwei RGD-Peptide unterschiedlicher Zell-Selektivität kovalent gekoppelt. Dabei wurden Acrylatpeptid 3, Typ iiia, mit einer Selektivität für alphaᵥbeta₃/alphaᵥbeta₅-Integrin-tragende Osteoblasten, und Acrylatpeptid 5, Typ iiia, mit einer Selektivität für alpha_{IIb}beta₃-positive Thrombozyten, wie für Beispiel 3 beschrieben an die PMMA-Formkörper gebunden.
**a.** Im ersten Teilversuch wurden PMMA-Formkörper mit den Acrylatpeptiden 3 und 5 in Konzentrationen in der Beschichtungslösung von jeweils 100 µM, 10 µM und 1 µM beschichtet. Anschließend wurden diese RGD-Peptid-beschichteten Materialien parallel mit Osteoblasten und Thrombozyten beimpft und deren Zelladhäsion bestimmt.
   Sowohl auf Acrylatpeptid 3- als auch auf Acrylatpeptid 5-beschichteten PMMA-Formkörpern wurden jeweils parallel MC3T3 H1-Osteoblasten (350.000 Zellen/cm²) bzw. humane Thrombozyten (50 Mio. Zellen/ cm²) angesät und deren Zelladhäsion, wie in Beispiel 3 beschrieben, bestimmt.
   Humane Thrombozyten wurden in Anlehnung an das Verfahren von Shattil und Brass (J. Biol. Chem. 262:992-1000, 1987) präpariert.
   Mit diesem Experiment konnte die Zellselektivität der beiden RGD-Peptide demonstriert werden.
   Auf Acrylatpeptid 3 (alphaᵥbeta₃-/alphaᵥbeta₅-selektiv)-beschichteten PMMA-Formkörpem adherierten bevorzugt alphaᵥbeta₃-/alphaᵥbeta₅-positive Osteoblasten im Vergleich zu alphaᵥbeta₃-/alphaᵥbeta₅-negativen Thrombozyten (Abb. 14). Dabei wurden für Osteoblasten kalkulierte Abs (405 nm) von ca. 6,0 (für 100 µM Peptid in der Beschichtungslösung), ca. 2,0 (für 10 µM Peptid) bzw. ca. 1,8 (für 1 µM Peptid) gefunden. Für Thrombozyten dagegen wurden signifikant niedrigere Zelladhäsionswerte von ca. 3,0 Abs (für 100 µM Peptid in der Beschichtungslösung), ca. 1,5 Abs (für 10 µM Peptid) bzw. ca. 0,2 Abs (für 1 µM Peptid) gefunden. Auf Acrylatpeptid 5 (alpha_{IIb}beta₃-selektiv)-beschichteten PMMA-Formkörpern dagegen wurde ein umgekehrter Effekt gefunden. Hier adherierten bevorzugt alpha_{IIb-}beta₃-positive Thrombozyten im Vergleich zu alpha_{IIb}beta₃-negativen Osteoblasten (Abb. 15).
   Dabei wurden für Thrombozyten kalkulierte Abs (405 nm) von ca. 4,0 (für 100 µM Peptid in der Beschichtungslösung), ca. 0,5 (für 10 µM Peptid) bzw. ca. 0,2 (für 1 µM Peptid) gefunden. Für Osteoblasten dagegen wurden signifikant niedrigere Zelladhäsionswerte von ca. 1,5 Abs (für 100 µM Peptid in der Beschichtungslösung), ca. 1,0 Abs (für 10 µM Peptid) bzw. ca. 0,2 Abs (für 1 µM Peptid) gefunden.
**b.** Im zweiten Teilversuch wurden ebenfalls PMMA-Formkörper mit den Acrylatpeptiden 3 und 5 beschichtet, allerdings in nur einer Konzentration in der Beschichtungslösung von jeweils 100 µM. Anschließend wurden diese RGD-Peptid-beschichteten Materialien parallel mit Osteoblasten, Thrombozyten bzw. einem Osteoblasten/Thrombozyten-Gemisch beimpft und deren Zelladhäsion bestimmt. Sowohl auf Acrylatpeptid 3- als auch auf Acrylatpeptid 5-beschichteten PMMA-Formkörpem wurden jeweils parallel MC3T3 H1-Osteoblasten (350.000 Zellen/cm²), humane Thrombozyten (50 Mio. Zellen/ cm²) bzw. ein Zell-Gemisch (350.000 Osteoblasten/cm² bzw. 50 Mio. Thrombozyten/cm²) angesät und deren Zelladhäsion, wie in Beispiel 3 beschrieben, bestimmt.

Auf Acrylatpeptid 3-beschichteten PMMA-Formkörpern adherierten bevorzugt alphaᵥbeta₃-/alphaᵥbeta₅-positive Osteoblasten (ca. 6,0 Abs) im Vergleich zu alphaᵥbeta₃-/alphaᵥbeta₅-negativen Thrombozyten (ca. 3,0 Abs) (Abb. 16). Wurden beide Zell-Species als Gemisch angesät, wurde ein Ergebnis erhalten, das demjenigen von Osteoblasten alleine entspricht (ca. 6,5 Abs).

Auf Acrylatpeptid 5 (alpha_{IIb}beta₃-selektiv)-beschichteten PMMA-Formkörpern dagegen wurde ein umgekehrter Effekt gefunden. Hier adherierten bevorzugt alpha_{IIb-} beta₃-positive Thrombozyten (ca. 4,0 Abs) im Vergleich zu alpha_{IIb}beta₃-negativen Osteoblasten (ca. 1,5 Abs) (Abb. 17). Wurden beide Zell-Species als Gemisch angesät, wurde ein Ergebnis erhalten, das etwa der Summe der Einzelwerte für Thrombozyten und Osteoblasten entspricht (ca. 6,5 Abs).

Diese Ergebnisse belegen, daß die Beschichtungen von Implantatoberflächen mit verschiedenen zellselektiven RGD-Peptiden zur Generation von Implantaten genutzt werden können, die die bevorzugte Adhäsion ausgewählter Zell-Species vermitteln. Dabei zeigt der Vergleich zwischen Osteoblasten und Thrombozyten, daß
- die Oberflächenbeschichtung mit integrin-selektiven RGD-Peptiden die Adhäsion derjenigen Zell-Species, die über eine komplementäre Integrin-Ausstattung verfügen, gegenüber Zellen, die diese speziellen Integrine nicht oder nur in geringem Umfang besitzen, deutlich stimuliert werden kann.
- dieser Effekt auch bei Verwendung von Zellgemischen, was dem in vivo-Zustand wesentlich näher kommt, erhalten bleibt.

## Patentansprüche

1. Implantat, geeignet für unterschiedliche menschliche und tierische Organe, bestehend im wesentlichen aus einer Trägermatrix und einer diese Matrix umhüllenden Peptid-Beschichtung, die zur zielgerichteten Adhäsionsstimulierung von menschlichen oder tierischen Körperzellen gleichartige oder verschiedene Peptide enthält, welche Sequenzen aufweisen, die Bindungsstellen auf den für die Adhäsion verantwortlichen Integrin-Rezeptoren auf humanen oder tierischen Zellen erkennen, wobei die Trägermatrix reaktive bindungsfähige Gruppen an ihrer Oberfläche aufweist, die dazu befähigt sind, mit entsprechenden funktionellen reaktiven Gruppen besagter Peptidschicht eine stabile kovalente Bindung einzugehen, **dadurch gekennzeichnet, dass** besagte auf der Oberfläche des Implantats angeordnete Peptide so ausgewählt und lokal angeordnet sind, dass sie aufgrund ihrer entsprechend unterschiedlichen strukturbedingten, zelladhäsionsstimulierenden Aktivität spezifisch dem natürlichen unterschiedlichen komplementären Integrinmuster der in der jeweiligen Regionen an sie angrenzenden Gewebezellen entsprechen, in die das Implantat eingebracht werden soll, wodurch ein lokal differenziertes, selektives bioaktives Beschichtungsmuster der Implantatoberfläche vorliegt.

2. Implantat nach Anspruch 1, **dadurch gekennzeichnet, daß** die besagten Peptide mit Hilfe von Ankermolekülen auf der Oberfläche des Implantats fixiert sind.

3. Implantat nach Anspruch 2, **dadurch gekennzeichnet, daß** die Ankermoleküle aus einer der folgenden Strukturen bestehen:
-CO-(CH₂)ₖ-X-SH, (i)
wobei X eine Einfachbindung oder -CO-NH-(CH₂)ₗ-,
k = 2 bis 12 und l = 2 bis 4 bedeutet;
-CO-(CH₂)ₘ-[NH-CO-(CH₂)ₙ]ₚ-NH-CO-CH=CH₂, (ii)
wobei m, n = 2 bis 8; p = 0 bis 2 bedeuten;
-(CO-CH₂-O-CH₂-CH₂-O-CH₂-CH₂-NH)_{q}-CO-(CH₂)ᵣ-NH-CO-CH=CH₂, (iii)
wobei q = 1 bis 3 und r = 2 bis 8 bedeutet.

4. Implantat nach Anspruch 3, **dadurch gekennzeichnet, daß** die Ankermoleküle aus einen der folgenden Strukturen ausgewählt werden:
-CO-CH₂-CH₂-SH; (ia)
-CO-CH₂-CH₂-CO-NH-CH₂-CH₂-SH; (ib)
-CO-(CH₂)₅-NH-CO-CH=CH₂; (iia)
-CO-(CH₂)₅-NH-CO-(CH₂)₅-NH-CO-CH=CH₂; (iib)
-CO-CH₂-O-CH₂-CH₂-O-CH₂-CH₂-NH-CO-(CH₂)₅-NH-CO-CH=CH₂; (iiia)
-(CO-CH₂-O-CH₂-CH₂-O-CH₂-CH₂-NH)₂-CO-(CH₂)₅-NH-CO-CH=CH₂ (iiib)

5. Implantat nach einen der Ansprüche 2 bis 4, **dadurch gekennzeichnet, daß** die Peptide über die Carboxyl-Gruppe des Ankermoleküls durch Amid-Bindung und die Ankermoleküle über die Mercapto- oder Acrylat-Gruppe an die Implantatoberfläche gebunden sind.

6. Implantat nach Anspruch 5, **dadurch gekennzeichnet, daß** ein solches Peptid-Ankermolekül an der Implantatoberfläche fixiert ist, so daß ein sterisch kritischer, absoluter Mindestabstand zwischen Zellerkennungsdomäne des Peptides und unschichteter Materialoberfläche von 2,5 bis 3,5 nm, vorliegt.

7. Implantat nach Anspruch 1 bis 6, **dadurch gekennzeichnet, daß** die Peptide an αᵥβ₃/αᵥβ₅ exprimierende Zellen zu binden vermögen.

8. Implantat nach einem der Ansprüche 1 bis 7, **dadurch gekennzeichnet, daß** es Peptide mit der Aminosäuresequenz RGD aufweist.

9. Implantat nach Anspruch 8, **dadurch gekennzeichnet, daß** es Peptide der Sequenz cyclo-RGDfK aufweist.

10. Implantat nach einem der Ansprüche 1 bis 9, **dadurch gekennzeichnet, daß** es zwischem den besagten Peptiden bzw. Peptid-Ankermolekülen und der Oberfläche der Trägermatrix eine haftvermittelnde Zwischenschicht und / oder verzweigte Moleküle, die einen oberflächenvergrößernden Effekt bewirken, aufweist.

11. Implantat nach einem der Ansprüche 1 bis 10, **dadurch gekennzeichnet, daß** die Trägermatrix ein entsprechendes Formstück aus Keramik, Polymermaterial, Metall ist oder ein strukturiertes Gebilde aus diesen Materialien darstellt, daß durch in vivo oder in vitro Besiedlung mit Zellen als biohybrides Organ ausgebildet werden kann.

12. Verfahren zur Herstellung von Implantaten gemäß der Ansprüche 1 bis 11, **dadurch gekennzeichnet, daß** man
*(i)* die Integrin-Rezeptor-Struktur des Zielgewebes, in welches das Implantat in vivo eingebracht werden soll, mittels eines in-vitro Testsystems bestimmt,
*(ii)* die besagten Peptide mit der entsprechenden komplementären Struktur auswählt bzw. synthetisiert und,
*(iii)* die besagten Peptide direkt oder über die besagten Ankermoleküle an die jeweilige, gegebenenfalls modifizierte Oberfläche des Implantats kovalent bindet, wobei die lokale Anordnung unterschiedlicher Peptide auf der Oberfläche des Implantats der jeweiligen zuvor festgestellten lokalen Anordnung der Zielzellen mit dem komplementären Integrinmuster entspricht, in die das Implantat eingebracht werden soll.

13. Verfahren nach Anspruch 12, **dadurch gekennzeichnet, daß** die in vitro-Bestimmung der Integrin-Rezeptor-Struktur des Zielgewebes mittels immunhistologisch wirksamer Antikörper erfolgt.

14. Verfahren nach Anspruch 12 oder 13, **dadurch gekennzeichnet, daß** die Kopplung der besagten Peptide bzw. der Peptid-Ankermoleküle an die Implantatoberfläche mittels für andere Beschichtungsarten bekannter Verfahren erfolgt.

## Claims

1. Implant, suitable for various human and animal organs, essentially consisting of a support matrix and a peptide coating surrounding this matrix which comprises, for targeted adhesion stimulation of human or animal body cells, identical or different peptides which contain sequences which recognise bonding sites on the integrin receptors responsible for adhesion on human or animal cells, where the support matrix has reactive bonding-capable groups on its surface which are capable of forming a stable covalent bond with corresponding functional reactive groups of the said peptide layer, **characterised in that** the said peptides arranged on the surface of the implant are selected and locally arranged in such a way that they, owing to their correspondingly different structure-related, cell adhesion-stimulating activity, correspond specifically to the natural different complementary integrin pattern of the tissue cells that are adjacent to them in the respective regions and into which the implant is to be introduced, resulting in the presence of a locally differentiated, selective, bioactive coating pattern of the implant surface.

2. Implant according to Claim 1, **characterised in that** the said peptides are immobilised on the surface of the implant with the aid of anchor molecules.

3. Implant according to Claim 2, **characterised in that** the anchor molecules consist of one of the following structures:
-CO-(CH₂)ₖ-X-SH, (i)
where X denotes a single bond or -CO-NH-(CH₂)ₗ-,
k = 2 to 12 and l = 2 to 4;
-CO-(CH₂)ₘ-[NH-CO-(CH₂)ₙ]ₚ-NH-CO-CH=CH₂, (ii)
where m, n = 2 to 8; p = 0 to 2;
-(CO-CH₂-O-CH₂-CH₂-O-CH₂-CH₂-NH)_{q}-CO-(CH₂)ᵣ-NH-CO-CH=CH₂, (iii)
where q = 1 to 3 and r = 2 to 8.

4. Implant according to Claim 3, **characterised in that** the anchor molecules are selected from one of the following structures:
-CO-CH₂-CH₂-SH; (ia)
-CO-CH₂-CH₂-CO-NH-CH₂-CH₂-SH; (ib)
-CO-(CH₂)₅-NH-CO-CH=CH₂; (iia)
-CO-(CH₂)₅-NH-CO-(CH₂)₅-NH-CO-CH=CH₂; (iib)
-CO-CH₂-O-CH₂-CH₂-O-CH₂-CH₂-NH-CO-(CH₂)₅-NH-CO-CH=CH₂; (iiia)
-(CO-CH₂-O-CH₂-CH₂-O-CH₂-CH₂-NH)₂-CO-(CH₂)₅-NH-CO-CH=CH₂ (iiib)

5. Implant according to one of Claims 2 to 4, **characterised in that** the peptides are bonded to the implant surface via the carboxyl group of the anchor molecule by amide bonding and the anchor molecules are bonded to the implant surface via the mercapto or acrylate group.

6. Implant according to Claim 5, **characterised in that** a peptide anchor molecule of this type is immobilised on the implant surface in such a way that a sterically critical, absolute minimum separation between cell recognition domain of the peptide and uncoated material surface of 2.5 to 3.5 nm exists.

7. Implant according to Claims 1 to 6, **characterised in that** the peptides are capable of bonding to αᵥβ₃-/αᵥβ₅ expressing cells.

8. Implant according to one of Claims 1 to 7, **characterised in that** it contains peptides having the amino acid sequence RGD.

9. Implant according to Claim 8, **characterised in that** it contains peptides having the sequence cyclo-RGDfK.

10. Implant according to one of Claims 1 to 9, **characterised in that** it has, between the said peptides or peptide anchor molecules and the surface of the support matrix, an adhesion-promoting interlayer and/or branched molecules which cause a surface area-increasing effect.

11. Implant according to one of Claims 1 to 10, **characterised in that** the support matrix is a corresponding shaped piece of ceramic, polymer material or metal or represents a structured formation of these materials which can be formed as biohybrid organ by in-vivo or in-vitro colonisation with cells.

12. Process for the production of implants according to Claims 1 to 11, **characterised in that**
*(i)* the integrin receptor structure of the target tissue into which the implant is to be introduced in vivo is determined by means of an in-vitro test system,
*(ii)* the said peptides having the corresponding complementary structure are selected or synthesised and
*(iii)* the said peptides are covalently bonded directly or via the said anchor molecules to the respective, optionally modified surface of the implant, where the local arrangement of different peptides on the surface of the implant corresponds to the respective pre-determined local arrangement of the target cells having the complementary integrin pattern into which the implant is to be introduced.

13. Process according to Claim 12, **characterised in that** the in-vitro determination of the integrin receptor structure of the target tissue is carried out by means of immunohistologically effective antibodies.

14. Process according to Claim 12 or 13, **characterised in that** the coupling of the said peptides or of the peptide anchor molecules to the implant surface is carried out by means of processes known for other types of coating.

## Revendications

1. Implant qui convient pour divers organes humains et animaux, constitué essentiellement par une matrice support et par un revêtement de peptides qui entoure cette matrice, qui comprend, pour une stimulation d'adhérence ciblée de cellules du corps humain ou du corps animal, des peptides identiques ou des peptides différents qui contiennent des séquences qui reconnaissent des sites de liaison sur les récepteurs d'intégrine responsables de l'adhérence sur des cellules humaines ou animales, où la matrice support comporte des groupes susceptibles d'une liaison réactive sur sa surface, qui sont susceptibles de former une liaison covalente stable avec des groupes réactifs fonctionnels correspondants de ladite couche de peptides, **caractérisé en ce que** lesdits peptides qui sont agencés sur la surface de l'implant sont sélectionnés et agencés localement de telle sorte que, du fait de leur activité de stimulation d'adhérence de cellule rapportée à une structure différente correspondante, ils correspondent de façon spécifique au motif d'intégrine complémentaire différent naturel des cellules de tissu qui leur sont adjacentes dans les régions respectives et au niveau desquelles l'implant doit être introduit, ce qui aboutit à la présence d'un motif de revêtement bioactif sélectif localement différencié de la surface d'implant.

2. Implant selon la revendication 1, **caractérisé en ce que** lesdits peptides sont immobilisés sur la surface de l'implant à l'aide de molécules d'ancrage.

3. Implant selon la revendication 2, **caractérisé en ce que** les molécules d'ancrage sont constituées par l'une des structures qui suivent:
-CO-(CH₂)ₖ-X-SH, (i)
où X représente une liaison simple ou -CO-NH-(CH₂)ₗ-,
k = 2 à 12 et l = 2 à 4;
-CO-(CH₂)ₘ-[NH-CO-(CH₂)ₙ]ₚ-NH-CO-CH=CH₂, (ii)
où m, n = 2 à 8; p = 0 à 2;
-(CO-CH₂-O-CH₂-CH₂-O-CH₂-CH₂-NH)_{q}-CO-(CH₂)ᵣ-NH-CO-CH=CH₂, (iii)
où q = 1 à 3 et r = 2 à 8.

4. Implant selon la revendication 3, **caractérisé en ce que** les molécules d'ancrage sont choisies parmi les structures qui suivent:
-CO-CH₂-CH₂-SH; (ia)
-CO-CH₂-CH₂-CO-NH-CH₂-CH₂-SH; (ib)
-CO-(CH₂)₅-NH-CO-CH=CH₂; (iia)
-CO-(CH₂)₅-NH-CO-(CH₂)₅-NH-CO-CH=CH₂; (iib)
-CO-CH₂-O-CH₂-CH₂-O-CH₂-CH₂-NH-CO-(CH₂)₅-NH-CO-CH=CH₂; (iiia)
-(CO-CH₂-O-CH₂-CH₂-O-CH₂-CH₂-NH)₂-CO-(CH₂)₅-NH-CO-CH=CH₂ (iiib)

5. Implant selon l'une des revendications 2 à 4, **caractérisé en ce que** les peptides sont liés à la surface d'implant via le groupe carboxyle de la molécule d'ancrage au moyen d'une liaison amide et les molécules d'ancrage sont liées à la surface d'implant via le groupe mercapto ou acrylate.

6. Implant selon la revendication 5, **caractérisé en ce qu'**une molécule d'ancrage de peptide de ce type est immobilisée sur la surface d'implant de telle sorte qu'une séparation minimum absolue stériquement critique entre un domaine de reconnaissance de cellule du peptide et une surface de matériau non revêtue de 2,5 nm à 3,5 nm existe.

7. Implant selon les revendications 1 à 6, **caractérisé en ce que** les peptides sont susceptibles d'une liaison sur des cellules d'expression αᵥβ₃-/αᵥβ₅

8. Implant selon l'une des revendications 1 à 7, **caractérisé en ce qu'**il contient des peptides comportant la séquence acide amino RGD.

9. Implant selon la revendication 8, **caractérisé en ce qu'**il contient des peptides présentant la séquence cyclo-RGDfK.

10. Implant selon l'une des revendications 1 à 9, **caractérisé en ce qu'**il comporte, entre lesdits peptides ou lesdites molécules d'ancrage de peptide et la surface de la matrice support, une intercouche de stimulation d'adhérence et/ou des molécules dérivées qui génèrent un effet d'augmentation d'aire de surface.

11. Implant selon l'une des revendications 1 à 10, **caractérisé en ce que** la matrice support est une pièce conformée en correspondance en une céramique, en un matériau de polymère ou en un métal ou représente une formation structurée de ces matériaux qui peut être formée en tant qu'organe biohybride au moyen d'une colonisation in vivo ou in vitro avec des cellules.

12. Procédé pour la fabrication d'implants selon les revendications 1 à 11, **caractérisé en ce que**:
*(i)* la structure de récepteur d'intégrine du tissu cible à l'intérieur duquel l'implant doit être introduit in vivo est déterminée au moyen d'un système de test in vitro;
*(ii)* lesdits peptides présentant la structure complémentaire correspondante sont sélectionnés ou synthétisés; et
*(iii)* lesdits peptides sont liés de façon covalente directement ou via lesdites molécules d'ancrage sur la surface modifiée optionnellement respective de l'implant où l'agencement local de différents peptides sur la surface de l'implant correspond à l'agencement local prédéterminé respectif des cellules cibles présentant le motif d'intégrine complémentaire au niveau desquelles l'implant doit être introduit.

13. Procédé selon la revendication 12, **caractérisé en ce que** la détermination in vitro de la structure de récepteur d'intégrine du tissu cible est mise en oeuvre au moyen d'anticorps efficaces immunohistologiquement.

14. Procédé selon la revendication 12 ou 13, **caractérisé en ce que** ledit couplage desdits peptides ou desdites molécules d'ancrage de peptide sur la surface d'implant est mis en oeuvre au moyen de procédés connus pour d'autres types de revêtements.
